# EUROPÄISCHE PATENTANMELDUNG

(11) **EP 3 699 792 A1**
(43) Veröffentlichungstag der Anmeldung: **26.08.2020**
(21) Anmeldenummer: 19158314.5
(22) Anmeldetag: 20.02.2019
(51) Int. Cl.: G06F 21/44, G06F 21/73, G06K 19/06, G06F 16/955, G16H 40/63, G16H 10/60

(54) **VERBRAUCHSEINHEIT MIT SICHERHEITSELEMENT, SICHERHEITSELEMENT UND VERFAHREN ZUR HERSTELLERSEITIGEN SICHERUNG EINES ZULAESSIGEN VERBRAUCHS**

(71) Anmelder: Fresenius Medical Care Deutschland GmbH, 61352 Bad Homburg (DE); Fresenius Medical Care AG & Co. KGaA, 61352 Bad Homburg (DE)
(72) Erfinder: Hellhund, Jonas, 60438 Frankfurt (DE); Lindemann, Robert, 65193 Wiesbaden (DE); Peters, Arne, 61352 Bad Homburg (DE); Fischer, Gerome, 99947 Weberstedt (DE)
(74) Vertreter: Schwarz, Claudia

(57) **Zusammenfassung**

Die vorliegende Erfindung betrifft eine Verbrauchseinheit (D) für eine Verbrauchsanwendung eines Verbrauchsprodukts, vorzugsweise in einem Medizinapparat, mit einem Sicherheitselement (110) zur Sicherung gegen Manipulation oder Missbrauch durch unberechtigte Dritte, wobei das Sicherheitselement eine, einen ersten herstellerseitigen Ident-Code materiell ausbildende, erste Ident-Kennzeichnung (ID-1-Label) umfasst; die erste Ident-Kennzeichnung (ID-1-Label) unlösbar mit der Verbrauchseinheit (D) verbunden und zur eindeutigen Identifizierung der Verbrauchseinheit (D), wie hinsichtlich einer Produkttype und/oder Produktverbrauchseigenschaft der Verbrauchseinheit (D), eingerichtet und mit einem, vorzugsweise nicht-zerstörungsfrei, lösbaren Verdeckungsmittel (150) teilweise oder vollständig verdeckt, vorzugsweise optisch verdeckt, ist. Ferner werden ein Verfahren zur Kennzeichnung einer Verbrauchseinheit (D), ein Verfahren zur Freigabe einer Verbrauchseinheit (D) in einer zugehörigen Verbrauchsvorrichtung sowie ein Verfahren zur Systemsteuerung beider oben genannten Verfahren vorgeschlagen. Desweiteren betrifft die Erfindung ein zugehöriges Steuersystem bzw. ein zugehöriges Computerprogramm zur Ausführung eines oben genannten Verfahrens sowie ein Sicherheitselement (110).

## Beschreibung

Die vorliegende Erfindung betrifft eine Verbrauchseinheit für eine Verbrauchsanwendung eines Verbrauchsproduktes, vorzugsweise in einem Medizinapparat, welche mit einem Sicherheitselement vorgesehen wird, sowie ein Sicherheitselement zum Aufbringen auf eine Verbrauchseinheit. Ferner betrifft die Erfindung ein Verfahren zur Kennzeichnung einer Verbrauchseinheit, ferner ein Verfahren zur Freigabe einer Verbrauchseinheit in einer zugehörigen Verbrauchsvorrichtung wie einem Medizinapparat sowie ein Verfahren zur Systemsteuerung beider oben genannten Verfahren. Desweiteren betrifft die Erfindung ein zugehöriges Steuersystem bzw. ein zugehöriges Computerprogramm zur Ausführung eines oben genannten Verfahrens. Insbesondere betrifft die vorliegende Erfindung eine Möglichkeit zur herstellerseitigen Sicherung einer Verbrauchseinheit gegen Manipulation oder Missbrauch durch unberechtigte Dritte sowie zur Absicherung anwendungskritischer Eigenschaften des Verbrauchsproduktes in der zugehörigen Verbrauchsvorrichtung gegen einen unzulässigen Verbrauch. Beispielsweise kann vorliegend an einem Verbrauchsort herstellerseitig abgesichert werden, dass eine spezifische Produkttype eines Dialysemoduls nur zulässig bzw. kompatibel, insbesondere nur für eine für einen Patienten spezifische Diagnose und/oder in einem zugehörigen Dialysegerät, eingesetzt und aktiviert wird.

Im Stand der Technik, insbesondere im Medizinsektor mit der Notwendigkeit der im klinischen Alltag unbedingt einzuhaltenden Sterilität, sind Verbrauchseinheiten, sogenannte "Disposables", vielfältiger Ausprägung und Bauart gebräuchlich. Mit dem Fortschritt der Medizin ist ferner bekannt, Möglichkeiten einer zumindest teil-automatisierten Überwachung und Sicherung der Bereitstellung bzw. des Verbrauchs solcher Verbrauchseinheiten vorzusehen, damit ein Patient jedenfalls nur eine passende bzw. genau die ärztlich jeweils angeordnete Behandlung erhält. Im Sinne einer zumindest teilweisen Automatisierung der Sicherungsmaßnahmen zur Vermeidung menschlicher Fehler sowie einer erhöhten Effizienz der klinischen Abläufe ist auch bekannt, technisch unter Zuhilfenahme von, vorzugsweise gerätelesbaren, Codes zu arbeiten.

So offenbart DE 10 2016 221 337 ein medizinisches Behandlungssystem mit einem medizinischen Behandlungsgerät, welches zur Auswahl einer Behandlung sowie zur Erzeugung eines Patienten- und eines Behandlung-bezogenen Anforderungscodes eingerichtet ist, sowie mit einem Therapiefreigabe-Gerät zum Empfang des Anforderungscodes, weiterhin mit einer Freigabecodeerzeugungseinrichtung. Das medizinische Behandlungsgerät entscheidet zumindest in Abhängigkeit vom Freigabecode und eventuell dem zuvor erzeugten Anforderungscode, ob und gegebenenfalls wie die angeforderte Behandlung freigegeben wird.

Ausgehend vom vorgenannten Stand der Technik ist es eine Aufgabe der vorliegenden Erfindung, hinsichtlich einzelner Verbrauchseinheiten eines Verbrauchsproduktes Möglichkeiten zur Sicherstellung einer ordnungsgemäßen bzw. spezifisch für einen jeweiligen Verbraucher zulässigen Verbrauchsanwendung am Verbrauchsort zu schaffen bzw. zu verbessern. Eine Möglichkeit von einem nicht-ordnungsgemäßen bzw. unzulässigen Verbrauch soll spezifisch erkannt werden und idealerweise im Weiteren noch im Vorfeld unterbunden werden. Aufgrund der zuletzt gestiegenen Vielfalt von patientenspezifischen, individuellen Behandlungsbedürfnissen bzw. Verschreibungen sowie aufgrund der gestiegenen Komplexität klinischer Abläufe in Behandlungszentren sowie im Alltag von, häufig ambulanten, Patienten sind zuletzt die Anforderungen an die Sicherstellung bzw. Überwachung eines zulässigen, sicheren Verbrauchs von Verbrauchseinheiten gestiegen.

Dabei besteht eine Aufgabe darin, eine auch aus Sicht des Herstellers als produktverantwortlichem Marktteilnehmer und Gewährleister lückenlose und/oder Missbrauchssichere Sicherungsmöglichkeit nach Verlassen der Verbrauchseinheit aus seinem direkten Einflussbereich, d.h. insbesondere ab Werk, zu schaffen. Bisher auf originalen Verbrauchseinheiten verwendete Codes weisen den Nachteil auf, dass sie von unberechtigt handelnden Dritten, also Tätern wie beispielsweise Produktfälschern, einfach ausgelesen und kopiert werden können. Sodann können solche unrechtmäßig erhaltenen Codes auf nichtautorisierten, ungeeigneten Fremdprodukten und/oder auf unzulässig wiederbenutzten originalen Verbrauchseinheiten angebracht und/oder zu einer unerlaubten Freigabe von Behandlungen bzw. zum Verbrauch eingerichteten Vorrichtungen, wie einem Medizingerät, benutzt werden. Somit liegt ein zu lösendes Problem in dem Vorbeugen von gezielter Manipulation und/oder Missbrauch, also in dem Schutz gegen eine Umgehungsmöglichkeit mit nicht unerheblichen Gefährdungsfolgen für den Verbraucher. Mit anderen Worten löst die Erfindung vornehmlich die Aufgabe, einen bestimmungsmäßigen, vorzugsweise einen für einen jeweiligen Verbraucher spezifischen, Verbrauch einer originalen Verbrauchseinheit zu gewährleisten sowie die Sicherstellung einer ausschließlich berechtigten Verwendung. Der Erfindung liegen zudem die weiteren Aufgaben zu Grunde, die Herstellung und Lieferkette von Verbrauchseinheiten, ferner noch die effiziente und fehlersichere Bereitstellung einer Verbrauchseinheit an einem Verbrauchsort zu verbessern und/oder die an einer Verbrauchsanwendung, insbesondere im Medizinsektor bei einer Behandlung eines Patienten, beteiligten Personen zu entlasten.

Diese Aufgaben werden durch Gegenstände nach den unabhängigen Ansprüchen gelöst. Vorteilhafte Ausführungsformen, Weiterbildungen oder Varianten sind Gegenstand der abhängigen Ansprüche, der Beschreibung und der Figuren.

Gemäß einem ersten Aspekt wird die Aufgabe durch eine Verbrauchseinheit für eine Verbrauchsanwendung eines Verbrauchsprodukts, vorzugsweise zum Verbrauch in einem Medizinapparat, mit einem Sicherheitselement zur Sicherung gegen Manipulation oder Missbrauch durch unberechtigte Dritte gelöst. Dabei umfasst das Sicherheitselement eine erste Ident-Kennzeichnung, welche einen ersten Ident-Code materiell ausbildet. Erfindungsgemäß ist die erste Ident-Kennzeichnung unlösbar mit der Verbrauchseinheit verbunden und zur eindeutigen Identifizierung der Verbrauchseinheit, wie hinsichtlich einer Produkttype und/oder Produktverbrauchseigenschaft der Verbrauchseinheit, eingerichtet. Die erste Ident-Kennzeichnung ist mit einem lösbaren Verdeckungsmittel teilweise oder vollständig verdeckt, vorzugsweise optisch verdeckt.

Der Begriff "Verbrauchseinheit" bezieht sich vorliegend auf eine jeweils einzelne Einheit bzw. ein individuelles Stück. Eine Verbrauchseinheit findet vorliegend einzeln ihre Nutzung bzw. ihren Verbrauch bei einem Verbraucher wie einem Patienten und/oder bei einem Anwender wie einem Arzt. Eine Verbrauchseinheit ist ein einzelner Teil aus einer größeren Gesamtheit eines Verbrauchsproduktes. "Verbrauchsprodukt" meint ein als solches meist in vielfacher Stückzahl hergestelltes und/oder im Markt beziehbares Produkt bzw. einen Artikel. Eine Verbrauchseinheit betrifft einerseits Einmalanwendungen, insbesondere einen Wegwerfartikel, sowie gegebenenfalls mit einer Umverpackung bzw. Sekundärverpackung eine, vorzugsweise sterile, Verpackungseinheit. Eine Verbrauchseinheit betrifft andererseits recyclierbare oder wiederverwendbare Verbrauchseinheiten für Mehrfachanwendungen wie beispielsweise Moduleinheiten, welche durch eine, meist herstellerseitige, Aufbereitung und/oder eine Reinigung und/oder eine Sterilisation und/oder eine funktionale Neuausrüstung nach Verbrauch erneut in einen originalen Zustand zu einem neuerlichen Verbrauch versetzt werden. Als Moduleinheiten sind beispielhaft Filtereinsätze, insbesondere Hohlfaser-Module, aufzufassen. Verbrauchseinheiten umfassen rein physikalisches Verbrauchszubehör und/oder Primärverpackungen bzw. Applikatoren, welche vorzugsweise medizinische, chemische und/oder pharmazeutische, Zubereitungen bzw. Formulierungen bzw. Dosierungen, vorzugsweise aller Aggregatzustände, enthalten. Primärverpackungen sind also alle Verpackungsmittel, die in direkten Kontakt mit dem eigentlichen Verbrauchsprodukt kommen, beispielsweise Folien, Gläser, Dosen, Deckel. Primärverpackungen bzw. Applikatoren können aus verschiedensten produktbeständigen Materialien gefertigt sein, beispielsweise aus Kunststoff wie Polyethylen, Polypropylen, Polystyrol, aus Metall wie Edelstahl und/oder Aluminium, aus Glas, aus Porzellan oder aus Verbundstoffen z.B. Mehrschichtfolien. Im Medizinsektor sind Primärverpackungen bzw. Applikatoren nicht selten mehrkompartimentiert zur Erhöhung des Haltbarkeit eines beinhalteten mehrkompartimentiert vorgehaltenen Verbrauchsproduktes ausgeführt. Verbrauchseinheiten, auch im Falle von einer Ausgestaltung als Primärverpackung wie einem Applikator, sind häufig einzeln umverpackt, beispielsweise mit einer transparenten Folie wie einem Folienbeutel, Sachets oder Flowpacks. Dabei stellt ein Umverpackungsmittel eine Sekundärverpackung dar, unabhängig davon, ob die mit ihr umverpackte Verbrauchseinheit eine Primärverpackung umfasst oder nicht.
Mit anderen Worten können die Verbrauchseinheiten einerseits als solche eigenständig einmalig verbraucht werden, wie beispielsweise Infusionsbeutel oder Kathetersets. Andererseits können die Verbrauchseinheiten, in Falle eines technischen Gesamtsystems, in einer zugehörigen Verbrauchsvorrichtung wie in einem Medizinapparat zu einer modulartigen Verwendung vorgesehen sein. Ein Beispiel für ein Gesamtsystem betrifft als Verbrauchseinheit ein Dialysemodul mit produktspezifischen Dialyse-Anwendungseigenschaften, insb. hinsichtlich der Stofftrennung, welches in einem passenden Dialysegerät eines Dialysezentrums für einen bestimmten Patienten als Verbraucher verwendet wird. Als Varianten von Dialyseanwendungen seien hier Hämodiafiltration, Hämodialyse, Hämofiltration genannt. Hohlfasermodule können solche Varianten durch entsprechende Fahrweisen bzw. mittels entsprechender Ausstattungen ihrer Hohlfasermembran abbilden. Unterschiedliche Fahrweisen können durch entsprechende Steuerung einer Maschine, in der das Hohlfasermodul verwendet wird, variiert werden. Ferner können Anschlüsse von Zu- und Ableitungen an der Maschine geändert werden. Ein weiteres Beispiel für einen Verbrauchsgegenstand ist eine Membran und/oder ein Filter zur Verwendung in einer Umkehrosmose-Anlage.

Insofern als die Erfindung auf eine Absicherung eines initialen Verbrauchs der Verbrauchseinheit bzw. im Falle einer Zubehöreinheit einer Inbetriebnahme, nur durch Berechtigte abzielt, ist es nicht erfindungswesentlich, ob ein vollständiger Abverbrauch bzw. ein Aufbrauchen der Verbrauchseinheit stattfinden. Somit sind ausdrücklich auch Ausführungsformen umfasst, bei welchen nach erstmaligem berechtigtem Verbrauchsbeginn ein Abbruch mit einem verbleibenden Rest bzw. eine Unterbrechung und gegebenenfalls eine geeignete Wiederaufnahme des Verbrauchs eintritt.

Wie oben erwähnt, umfasst erfindungsgemäß das Sicherheitselement eine erste Ident-Kennzeichnung, welche einen ersten Ident-Code materiell ausbildet. Sowohl ein Ident-Code einerseits als auch eine jeweils zugehörige Ident-Kennzeichnung andererseits dienen als Identifikatoren für dieselbe Verbrauchseinheit. Sprich, beide Begriffe entsprechen einander, wobei lediglich im Sinne einer besseren Klarheit nach der jeweiligen Gestalt differenziert werden soll, also ob virtuell bzw. informationstechnisch oder ob materiell bzw. greifbar bzw. physikalisch. Beispielsweise handelt es sich bei einem Ident-Code um eine für die einzelnen Verbrauchseinheiten des Verbrauchsproduktes in der Herstellung fortlaufende Seriennummer, für welche ein individuell zugeordneter Code vergeben wird bzw. welche in eine verschlüsselte und/oder kodierte Form umgewandelt werden kann. Dies dient einer eindeutigen Identifizierung der individuellen Verbrauchseinheit anhand ihrer zugehörigen Ident-Kennzeichnung, welche den meist numerisch, also im virtuellen Raum vorliegenden Ident-Code im materiellen Raum bzw. körperlich ausbildet, insbesondere optisch darstellt bzw. (aus)lesbar abbildet. Der Begriff "Ident-Kennzeichnung" (englisch: "label") meint vorliegend eine Kenntlichmachung bzw. und eine Schaffung einer eindeutigen Identifizierungsmöglichkeit, insbesondere eine Beschriftung, ein Schild, ein Etikett, einen Aufkleber, einen Aufdruck, einen Stempel, eine Signatur, einen Transponder, einen Chip, usw. Dabei handelt es sich um eine für einen außenstehenden Dritten seitens des Informationsgehaltes nicht weiterführende, nicht einsehbare Form der herstellerseitigen Ident-Kennzeichnung.

Unter dem Begriff "Ident-Code" bzw. "Ident-Kennzeichnung" sollen im Rahmen der Erfindung alle im Stand der Technik bekannten Codes und insbesondere typische Standards verwendet werden können, aber auch zukünftige. Letzteres kann im zeitlichen Wandel mit neuen Code-Technologien wie Code-Generierungs- und/oder Code-Erfassungsmethoden gemäß zugrundeliegenden physikalischen Prinzipien erfolgen. Es sollen also die Begriffe "Ident-Code" bzw. "Ident-Kennzeichnung" vorliegend sinngemäß und insbesondere funktional verstanden sein. Was optisch sichtbare Ident-Kennzeichnungen anbelangt, sind beispielsweise einzeilige oder mehrzeilige sogenannte Barcodes bzw. Strichcodes etabliert. Vorzugsweise kann es sich um einen sogenannten "Codablock" (einen international praktisch angewendeten zweidimensionalen Strichcode) handeln. Besonders bevorzugt ist eine Kennzeichnung gemäß dem als spezielle Form des Barcodes für medizinische Anwendungen bekannten HIBC-Standard (HIBC: offizielle Abkürzung für Health Industry Barcode; verschiedene Standards der automatischen Datenkommunikation, die weltweit die sichere Produktidentifikation und Rückverfolgung in der Gesundheitsindustrie sicherstellen sollen). Eine andere zum Zeitpunkt der Erfindung praktisch übliche Ident-Kennzeichnung ist als "RFID-Label" eingeführt (englisch: radio-frequency identification, also "Identifizierung mit Hilfe elektromagnetischer Wellen") und bezeichnet eine Technologie für Sender-Empfänger-Systeme zum automatischen und berührungslosen Identifizieren und Lokalisieren von Objekten auf Basis von Radiowellen.

Wie oben erwähnt, ist erfindungsgemäß die erste Ident-Kennzeichnung unlösbar mit der Verbrauchseinheit verbunden und zur eindeutigen Identifizierung der Verbrauchseinheit, wie hinsichtlich einer Produkttype und/oder Produktverbrauchseigenschaft der Verbrauchseinheit, eingerichtet. Mit anderen Worten umfasst die erste Identifizierung der Verbrauchseinheit gemäß der ersten Ident-Kennzeichnung bzw. dem ersten Ident-Code eine Vielfalt von für sie relevanten Charakteristika und/oder Produktstammdaten, insbesondere technische Produktdaten und sonstige Informationen wie herstelltechnische und/oder anwendungstechnische Spezifkationen, eine Modellserie, eine individuelle Herstellzeit bzw. Produktionsschicht, einen Herstellort, eine Produktionsstätte und/oder (eine) Herstellanlage(n), einen oder mehrere Rohstoffe bzw. Halbfertigzeuge, eine Chargennummer, eine Prüfziffer, ein Prüßmeßprotokoll usw. Anwendungstechnische Produktverbrauchseigenschaften können z.B. Daten in Bezug auf Dialysierflüssigkeiten (Art und Zusammensetzung der Dialysierflüssigkeit und/ oder Substitutionsflüssigkeit), Dialysatoren, verwendete Einmalartikel wie Schlauchsets, Dialysefilter, usw. sein. Im Allgemeinen umfassen die herstelltechnischen und/oder anwendungstechnischen Daten zumindest so viele charakteristische Merkmale, dass eine Verwendung oder Behandlung eindeutig einem Verbraucher wie einem Patienten zuzuordnen ist. In dem hier verwendeten Sinn entspricht die als erste Identifizierung fungierende erste Ident-Kennzeichnung quasi einer 'Personalausweisnummer' oder einem Fingerabdruck für eine jeweilige Verbrauchseinheit. Die erste Identifizierung ist gedacht, um eine Identifizierbarkeit für die Verbrauchseinheit von einem Beginn in Form einer Identitätsvergabe an sie bis zu einem Ende in Form ihres Verbrauchs, zu schaffen. Ferner kann die erste Identifizierung im Sinne einer zeitlich noch darüberhinaus gehenden Nutzung der Information beispielsweise zur Speicherung von weiterführenden Prozessen dienen.

Wie oben erwähnt, ist erfindungsgemäß die erste Ident-Kennzeichnung mit einem lösbaren Verdeckungsmittel teilweise oder vollständig verdeckt, vorzugsweise optisch verdeckt. Durch die zumindest teilweise Verdeckung der herstellerseitig vorgesehenen ersten Ident-Kennzeichnung wird bewirkt, dass diese in ihrem von dem Verdeckungsmittel verdeckten Teil für Dritte, insbesondere für Unberechtigte, nicht erkennbar bzw. nicht erfassbar wird. Sofern ein Teil der ersten Ident-Kennzeichnung unverdeckt bleibt, kann der offen zugängliche Teil in üblicher Weise von jedermann entlang der Wertschöpfungskette, vom Hersteller, beispielsweise in einem Laborjournal und/oder Logbuch, bis zu einem Anwender und/oder Verbraucher und gegebenenfalls auch von dezentralen weiteren Teilnehmern und/oder in einer Patientenakte verwendet werden. Unter dem Begriff "Verdeckungsmittel" sind alle zum Verdecken, zum Unsichtbarmachen, zur Unkenntlichmachung und/oder zur Unverwendbarkeit der Ident-Kennzeichnung (in dem abgedeckten Bereich) geeigneten Mittel zu verstehen, beispielsweise ein Aufkleber, eine aufgebrachte Deckschicht, eine Folie und/oder ein Aufdruck, insbesondere eine optische Abdeckung, und/oder im Falle einer Transponder-Kennzeichnung ein Störsignaltransponder oder Mittel zum Verstimmen eines Resonanzkreises zu verstehen.

Dabei kann es bevorzugt sein, dass das Verdeckungsmittel nicht-zerstörungsfrei lösbar von der ersten Ident-Kennzeichnung ausgebildet ist. Der Begriff "nicht-zerstörungsfrei" bezieht sich lediglich auf das von der ersten Ident-Kennzeichnung zu lösende bzw. wieder zu trennende Verdeckungsmittel, jedoch gerade nicht auf die Ident-Kennzeichnung, deren Integrität durch Rückgängigmachen des Verdeckens, zumindest weitestgehend, unberührt im Sinne der weiteren Verwendbarkeit bleiben muss. Gemeint ist, dass das Verdeckungsmittel, insbesondere ein materiell gestaltetes Verdeckungsmittel, in dem bzw. durch den Lösevorgang seine weitere funktionale Verwendbarkeit verliert. Durch eine bevorzugte nichtzerstörungsfreie Ausgestaltung wird bewirkt, dass ein Ablösen des Verdeckungsmittels irreversibel bzw. nur einmalig möglich ist. Mit anderen Worten bewirkt ein Ablösen, dass ein Wiederanbringen des Verdeckungsmittels nicht möglich ist. Beispielsweise kann dies in Form eines abziehbaren Aufklebers mit mindestens einer Sollbruchstelle wie einer Perforation und/oder in Form eines Rubbelfeldes ausgestaltet sein. Es kann alternativ oder kumulativ ausreichend sein, dass eine zum Verdecken notwendige Haft- bzw. Klebestärke und/oder die innere materielle Struktur des Verdeckungsmittels nach einmaligem Lösen derart stark herabgesetzt ist, dass eine Reposition des Verdeckungsmittels missglücken müsste und/oder zumindest gut erkennbar bzw. feststellbar, idealerweise auffällig, wäre.

Insbesondere kann ein teilweise unverdeckter, insbesondere ein optisch einfach (aus)lesbarer, Teil der ersten Ident-Kennzeichnung zu Logistikzwecken wie der Nachverfolgbarkeit der Verfahrensschritte und der Transportwege (englisch: "Tracking" oder "Traceability") entlang der Wertschöpfungskette (englisch: "Supply Chain") dienen. Nach Verdecken kann im weiteren ein teilweise unverdeckter Teil der ersten Ident-Kennzeichnung zur eindeutigen Dokumentation in gewohnter Weise verwendet werden, beispielsweise im Rahmen der im Medizin- und Pharmasektor erforderlichen sogenannten "Guten Herstellungspraxis" (englisch: Good Manufacturing Practice, abgekürzt GMP). Darunter versteht der Fachmann Richtlinien zur Qualitätssicherung und Auditierung der Produktionsabläufe und -umgebung in der Produktion primär, aber nicht ausschließlich, von Arzneimitteln und Wirkstoffen (siehe insb. die zur Zeit geltende gesetzliche EG-Richtlinie 2003/94/EG zur Festlegung der Grundsätze und Leitlinien der Guten Herstellungspraxis für Humanarzneimittel; ferner die ISO 9001-Norm zur Auditierung von Qualitätsmanagement-Systemen; in den USA die Auflagen der amerikanischen FDA-Behörde).

In einer Ausführungsform bildet sowohl der durch das Verdeckungsmittel verdeckte als auch der unverdeckte Teil der ersten Ident-Kennzeichnung jeweils separat einen sinnvollen graphischen Ident-Code aus.

Im besonders bevorzugten Falle einer ersten Ident-Kennzeichnung in Form des oben genannten bevorzugten "Codablocks" und/oder bei einer mehrzeiligen Ausführung kann eine nicht vollständige und nur teilweise Verdeckung besonders bevorzugt derart erfolgen, dass nur einige der vorgesehenen Zeilen von dem Verdeckungsmittel verdeckt werden, während die weiter sichtbaren Zeilen als unverdeckter Teil ab Kennzeichnung von jedermann genutzt werden können. Bei dieser quasi integral gestalteten bevorzugten Ausführungsform handelt es sich um eine besonders effiziente Umsetzung der Erfindung, indem unter anderem auch eine fortgeführte Kompatibilität zu bereits bestehenden logistischen Einrichtungen auf besonders einfache Weise ausgeführt wird.

Bei einer weiterhin bevorzugten Ausführungsform sind der verdeckte und der unverdeckte Teil der ersten Ident-Kennzeichnung nach dem industriellen Standard "Code 49" dargestellt. "Code 49" meint eine für einen mehrreihigen Barcode für erhöhte Sicherheitsanforderungen wie in der Luftfahrt eingeführte Bezeichnung, umfassend eine sogenannte Parity-Reihe, mittels welcher für jedes Zeichen die Parität überprüft werden kann. Zum "Code 49" wird ferner auf die Offenbarung der bei der "Association for Automated Identification and Mobility" verfügbaren genauen "Codespezifikation" verwiesen. In dieser Ausführungsform wird vorliegend zumindest die letzte Zeile, welche die Prüfzeichen für den gesamten Block enthält, mit einer einen anderen Inhalt codierenden Zeile überklebt und die Prüfziffer für den überklebten Block entsprechend zur Erhaltung der Code-Integrität der Kennzeichnung angepasst.

Bei einer noch weiteren bevorzugten Ausführungsform sind der verdeckte und der unverdeckte Teil der ersten Ident-Kennzeichnung in einem sogenannten "Composite Code" kodiert, was vorliegend bedeuten soll, dass in den beiden Teilen jeweils eine Verknüpfung bzw. ein Querverweis bzw. ein Link zum anderen Teil mitcodiert sein kann. Beispielsweise kann der Composite-Code als ein Doppelcode-Symbol ausgeführt sein, wie in Form eines sich aus einem linearen, also 1-dimensionalen Strichcode und einem zusätzlichen 2-dimensionalen Code zusammensetzten Strichcodes.

Im Gegensatz zu einer nur teilweisen Verdeckung kann in einer anderen Ausführungsform gerade eine vollständige Verdeckung der ersten Ident-Kennzeichnung erwünscht sein, um eine versteckte Ident-Kennzeichnung zu bewirken. Eine solche verborgene Gestaltung bewirkt besonderen Schutz gegen Fälschungsaktivitäten unberechtigter Dritte. Insbesondere kann die erste Ident-Kennzeichnung an im Originalzustand der Verbrauchseinheit unzugänglicher Position vorgesehen sein.

In einer technisch vorteilhaften Ausführungsform der Verbrauchseinheit umfasst diese weiterhin eine, einen zweiten Ident-Code materiell ausbildende, zweite Ident-Kennzeichnung. Dabei ist die zweite Ident-Kennzeichnung nicht-verdeckt, vorzugsweise optisch sichtbar, und zu einer zumindest vorübergehenden logistischen Verwendung, wie Nachverfolgbarkeit, eingerichtet. Diese Ausführungsform umfasst als mögliche Varianten, dass die zweite Ident-Kennzeichnung nicht ausschließlich oder gar nicht auf der einzelnen, gegebenenfalls einzeln original umverpackten z.B. folienverpackten, Verbrauchseinheit selber angebracht ist, sondern auf einer Mehrzahl von Verbrauchseinheiten wie einer Doppel-, Mehrfach-, bzw. Großpackung und/oder einem Transportgebinde, z.B. einer Kartonage usw.

Vorzugsweise ist die zweite Ident-Kennzeichnung ein Teil des Verdeckungsmittels, sprich, es entfällt ein separates Verdeckungsmittel zur Verdeckung der ersten Ident-Kennzeichnung, insofern als die Verdeckung der ersten Ident-Kennzeichnung direkt durch die zweite Ident-Kennzeichnung erfolgt. Ein Teil des Verdeckungsmittels kann auch heißen, dass materielle Identität besteht, sprich, dass die zweite Ident-Kennzeichnung und das Verdeckungsmittel als Einheit zusammenfallen. Beispielsweise kann dies in Form von zwei übereinander aufgebrachten Schichten aus einer ersten und einer zweiten Ident-Kennzeichnung erfolgen. In dieser bevorzugten Ausführungsform kann es weiter bevorzugt sein, dass der unverdeckte Teil der mit der zweiten Ident-Kennzeichnung verdeckten ersten Ident-Kennzeichnung und die gänzlich unverdeckte zweite Ident-Kennzeichnung wiederum zusammengenommen eine dritte Ident-Kennzeichnung mit einem sinnvollen zugehörigen, aus der Überlagerung entstehenden, dritten Ident-Code darstellen. Dadurch können besondere Fehlerkorrekturmechanismen der Kennzeichnung bzw. der Codierung vorgenommen werden. Alternativ können Teile des verdeckten Teils der ersten Ident-Kennzeichnung mit der Farbe des darunterliegenden Untergrunds quasi 'gelöscht' werden. Dies erlaubt erhöhte Freiheitsgrade bei der Codierung des nicht-verdeckten Teils der ersten Ident-Kennzeichnung.

Gemäß einem zweiten Aspekt der vorliegenden Erfindung wird ein Verfahren zur Kennzeichnung einer Verbrauchseinheit für eine Verbrauchsanwendung eines Verbrauchsprodukts, vorzugsweise zum Verbrauch in einem Medizinapparat, zur Sicherung gegen Manipulation oder Missbrauch durch unberechtigte Dritte, vorgeschlagen. Im Sinne einer Vermeidung von Wiederholungen wird auf die zuvor getroffenen Begriffsdefinitionen verwiesen. Es ergeben sich die Merkmale, Merkmalskombinationen und die sich aus diesen ergebenden Vorteile entsprechend denjenigen, welche zuvor in Verbindung mit dem erstgenannten Erfindungsaspekt ausgeführt worden sind. In einem ersten Schritt wird ein erster Ident-Code informationstechnisch generiert bzw. rechentechnisch erzeugt und durch einen Hersteller des Verbrauchsproduktes gespeichert. Damit liegt der Ident-Code auf einem Speichermedium, insbesondere in einer Datenbank, in dem Einflussbereich des Herstellers gespeichert vor, welcher die Zugriffsrechte auf die Daten verwaltet und überwacht, insofern intern und vertraulich halten kann. In einem nachfolgenden Schritt wird die der Verbrauchseinheit mit einer, den ersten Ident-Code materiell ausbildenden, ersten Ident-Kennzeichnung gekennzeichnet. Mit diesem Schritt geht ein weiterer Schritt einher, um eine eindeutige Identifizierungsmöglichkeit der Verbrauchseinheit mit ihren für einen späteren Verbraucher und/oder Anwender relevanten Charakteristika und Merkmalen zu bewirken. Und zwar erfolgt parallel auf informationstechnischer Ebene ein Zuordnen von einer Produkttype und/oder Produktverbrauchseigenschaft der Verbrauchseinheit zu dem ersten Ident-Code, wodurch eine erste Datenrelation gebildet wird. Diese Transaktion bzw. Information über den Schritt, insb. die erste Datenrelation, wird durch den Hersteller gespeichert. Mit anderen Worten werden dem Produktionsprozess und/oder dem Logistikprozess zugeordnete Daten z.B. in Form von Datenpaketen (Stichwort "Digitale Fabrik"), die Informationen darüber repräsentieren, welche Verbrauchseinheit sich durch welche Produkttype und/oder Produktverbrauchseigenschaft anwendungstechnisch relevant charakterisieren lässt, in dem Schritt der ersten Kennzeichnung miteinander verknüpft bzw. in einer Funktion informationstechnisch zugeordnet bzw. hinter der ersten Ident-Kennzeichnung auf Ident-Code-Ebene hinterlegt. Diese eindeutige Informationsverknüpfung im Zuge der ersten Kennzeichnung dient dazu, dass zu jedem, auch weit späteren, Zeitpunkt und auch an einem anderen Ort über den ersten Ident-Code die unter ihm zu der jeweiligen Verbrauchseinheit hinterlegten charakteristischen Informationen, also bspw. eine Produkttype und/oder Produktverbrauchseigenschaft, genau über eine Verwendung dieser ersten Datenrelation wieder herangezogen und verfügbar gemacht werden können. Insofern als die erste Datenrelation im Besitz des Herstellers liegt, kann ein solcher Umkehrschritt bzw. die Schlussfolgerung von dem ersten Ident-Code auf die in der ersten Datenrelation verknüpften Informationen nur unter Einbindung des Herstellers, also mit Berechtigung durch ihn, stattfinden.

Erfindungsgemäß erfolgt in einem weiteren Schritt ein Verdecken der ersten Ident-Kennzeichnung mit einem Verdeckungsmittel. Dabei wird die erste Ident-Kennzeichnung teilweise oder vollständig verdeckt, vorzugsweise optisch verdeckt. Ferner wird dabei das Verdeckungsmittel lösbar, von der ersten Ident-Kennzeichnung aufgebracht. Mit dieser Merkmalskombination wird bewirkt, dass bis zu einem Entfernen des wieder lösbaren Verdeckungsmittels die erste Ident-Kennzeichnung für eine Nutzung verdeckt bleibt, somit das oben erwähnte Heranziehen der ersten Datenrelation über den Ident-Code bis zu diesem Zeitpunkt durch niemanden erfolgen kann.

Beispielsweise werden beim Verdecken der ersten Ident-Kennzeichnung lediglich einzelne graphische Elemente der Code-Darstellung mit einer Farbe überdeckt, weiter bevorzugt auf einem ablösbaren Träger, so dass die unverdeckten graphischen Elemente eine neue, sinnvolle Ident-Code darstellende Ident-Kennzeichnung bilden.

Insbesondere kann eine vollständige Verdeckung vorteilhaft sein, wenn das Verdeckungsmittel und unter ihr die erste Ident-Kennzeichnung in einer geeigneten diskreten Ausgestaltung der Verbrauchseinheit gänzlich verborgen bleibt, um Unberechtigte wie Fälscher gar nicht erst auf den Gedanken etwaiger Nutzungsversuche nach irgendwelcher Art von Manipulation zu bringen.

Alternativ kann gerade eine teilweise Verdeckung besonders bevorzugt sein, um eine besonders effektive, quasi integrale Ausführungsform eines Sicherheitselementes für eine Verbrauchseinheit darzustellen. Denn bei einer teilweisen Verdeckung, beispielsweise von einem Teilbereich eines mehrzeiligen Strichcodes wie einem Codablock-Standard, entsteht eine besonders vorteilhafte Kombination eines ersten verdeckten Teilbereiches der ersten Ident-Kennzeichnung und eines zweiten unverdeckten Teilbereiches der ersten Ident-Kennzeichnung. Insofern kann der unverdeckte Teilbereich der ersten Ident-Kennzeichnung zur Ausbildung bzw. graphischen Darstellung eines separat enthaltenen Ident-Codes, insbesondere zu den für Ident-Codes üblichen Zwecken, genutzt werden, insbesondere Handling und Tracking. Dabei bildet der verdeckte Teilbereich der ersten Ident-Kennzeichnung, für sich alleine genommen und/oder in Zusammenschau mit dem unverdeckten Teilbereich, den zur Identifikation und Sicherung herangezogenen ersten Ident-Code aus.

Vorzugsweise ist das Verdeckungsmittel nicht-zerstörungsfrei lösbar ausgestaltet. Das dient dazu, dass - wie bei einem Siegellack auf einem Brief - ein Sichtbarmachen der ersten Ident-Kennzeichnung nur einmalig erfolgen kann und im weiteren als Nicht-Originalzustand für jedermann offenkundig erkennbar ist.

Je nachdem können aus diversen technischen Erwägungen, insbesondere aus praktischen Gründen hinsichtlich des Layouts der Produktionsfabrik sowie der konkret eingesetzten Herstellungsanlagen, aber auch hinsichtlich des Materialflusses und der Logistik einschließlich der Lagerhaltung, unterschiedliche Varianten der Verfahrensschritte erstes Kennzeichnen und Verdecken in der bzw. in den entsprechenden Verfahrensstufe(n) bevorzugt sein: Einerseits kann das Verdecken nachfolgend zum ersten Kennzeichen stattfinden, beispielsweise in einer getakteten, linearen Kontianlage mit Förderband durch Aufbringen einer ersten Ident-Kennzeichnung in einer vorgelagerten Anlagenstufe und dann Aufbringen eines Verdeckungsmittels in einer darauf folgenden weiteren Anlagenstufe. Andererseits kann es bevorzugt sein, im Schritt der ersten Kennzeichnung auf die Verbrauchseinheit eine bevorzugte Ausführungsform eines vorgefertigten Sicherheitselement aufzubringen. Das vorgefertigte Sicherheitselement umfasst eine Kombination einer bereits mit einem Verdeckungsmittel zuvor verdeckten Ident-Kennzeichnung, beispielsweise eine Verbundschicht. In einer solchen oben beschriebenen kontinuierlichen Herstellprozessführung kann das vorgefertigte Sicherheitselement in der Herstelllinie zunächst in einer vorgelagerten Stufe hergestellt und/oder aus einem Lager an die Herstellerlinie zugeführt werden.

Vorliegend ist mit dem Begriff "Hersteller" (bzw. "herstellerseitig") eine Firma gemeint, welche die spezifischen Eigenschaften ihres Verbrauchsproduktes in dessen Anwendung beim Verbraucher gewährleisten möchte bzw. gesetzlich zu gewährleisten hat. Insbesondere kann es sich im Falle von auditierungspflichtigen Prozessen, beispielsweise im Bereich der Medizinprodukte, bei dem Hersteller um eine zur Herstellung bzw. zum Vertrieb des Verbrauchsproduktes qualifizierte, eine berechtigte bzw. eine seitens zu involvierender Behörden zugelassene Firma handeln. Der Begriff umfasst in seinem örtlichen Sinne die nationalen und internationalen Standorte der Herstellung, der Lagerhaltung und/oder der Logistik. Gleichfalls sind im Rahmen der vorliegenden Erfindung nicht nur Fertigungsbereiche, sondern auch primäre und sekundäre Verpackungslinien umfasst. Desweiteren betrifft der Begriff vorliegend nicht nur den ursprünglichen Hersteller im engsten Sinne, sondern auch einen Vertragspartner und/oder einen Subunternehmer und/oder einen zertifizierten Lohnhersteller und/oder einen Logistikdienstleister, welche im Auftrag der und/oder für die Ursprungsfirma handeln. So kann es der Fall sein, dass eine herstellende und/oder verpackende Geschäftsprozessstufe vorab bzw. die Auslieferung bzw. der Versand der Waren in Gestalt der vorliegenden Verbrauchseinheiten ab Werk und/oder Warenlager (englisch: dem sogenannten "Dispatch") hin zu den Verbrauchern bzw. Anwendern an extern in qualifizierter Weise vergeben (englisch: "outgesourced") wird. Hinsichtlich einer Dienstleistung der Informationstechnologie an den Hersteller sind solche, vornehmlich vertragsgebundenen, Dienstleistungsunternehmen gleichermaßen vom Begriff "herstellerseitig" umfasst. Beispielsweise soll im Rahmen der Erfindung der Unterfall mitumfasst sein, dass ein Ident-Code durch einen separaten Informationstechnologiedienstleister im Vorfeld erzeugt und an den Hersteller zu seiner Verwendung weitergegeben werden.

Der Begriff "informationstechnisch" ist als Fachbegriff zu verstehen und bezieht sich auf die computer-basierte oder -implementierte Ausführung von Schritten, die auch mittels Software und/oder Hardware implementiert sein können. Ein informationstechnisches Erzeugen und Zuordnen können z.B. durch entsprechende Algorithmen und/oder Applikationen ausgeführt werden. Dabei können die jeweiligen Algorithmen und/oder Applikationen lokal und/oder remote (z.B. auf einer in Datenaustausch stehenden Computerinstanz) ausgeführt werden. Zudem können die entsprechenden Algorithmen und/oder Applikationen in virtualisierter Form bereitgestellt werden. "Informationstechnisch" bezieht sich somit auf eine automatische und letztendlich Prozessor-basierte Implementierung unter Verwendung von integrierten Schaltkreismodulen.

Dem Begriff "Datenrelation" liegt eine mathematische Abbildungsvorschrift (bzw. Funktion, Zuordnung) zugrunde. Es kann vorliegend sich um eine bijektive oder eineindeutige Datenrelation handeln. Wesentlich ist, dass insbesondere ausgehend von dem ersten Ident-Code eine Identifizierung, insbesondere ein Umkehrschluss auf eine für die Freigabe anwendungsrelevante Produkttype bzw. Produktverbrauchseigenschaft ermöglicht bzw. eingerichtet ist.

In einer technisch vorteilhaften Ausführungsform umfasst das Verfahren zur Kennzeichnung weitere Schritte: ein informationstechnisches Generieren eines zweiten Ident-Codes und Speichern durch den Hersteller; Kennzeichnen der Verbrauchseinheit mit einer, den zweiten Ident-Code materiell ausbildenden, zweiten Ident-Kennzeichnung, wobei die zweite Ident-Kennzeichnung nicht-verdeckt, vorzugsweise optisch sichtbar, aufgebracht wird; und informationstechnisches Zuordnen des zweiten Ident-Codes zu dem ersten Ident-Code zur Bildung einer zweiten Datenrelation und Speichern durch den Hersteller.

Vorzugsweise können der erste Ident-Code und der zweite Ident-Code als Teile eines informationstechnisch übergeordneten weiteren Ident-Codes generiert sein. Alternativ kann es gerade auch bevorzugt sein, dass der erste Ident-Code und der zweite Ident-Code gänzlich unabhängig voneinander sind. Es können der erste Ident-Code und der zweite Ident-Code auf unterschiedliche Weise generiert werden. Unabhängig davon können die erste Ident-Kennzeichnung und die zweite Ident-Kennzeichnung nach unterschiedlicher Art bzw. gemäß anderem technischem Prinzip ausgestaltet und/oder auf unterschiedliche Weise auf die Verbrauchseinheit aufgebracht werden. Beispielsweise könnte eine erste Ident-Kennzeichnung als Barcode auf die Verbrauchseinheit aufgedruckt und die zweite Ident-Kennzeichnung in Form eines RFID-Labels aufgeklebt werden.

In einer technisch vorteilhaften Ausführungsform des Verfahrens zur Kennzeichnung ist die zweite Ident-Kennzeichnung zum Kennzeichnen der Verbrauchseinheit ein Teil des Verdeckungsmittels. Ein Teil des Verdeckungsmittels kann auch heißen, dass materielle Identität besteht, sprich, dass die zweite Ident-Kennzeichnung und das Verdeckungsmittel als Einheit zusammenfallen. Damit wird auch das teilweise oder vollständige Verdecken der ersten Ident-Kennzeichnung in sehr effizienter Weise bewirkt, indem auf ein zusätzliches Verdeckungsmittel verzichtet werden kann.

In einer technisch vorteilhaften Ausführungsform des Verfahrens zur Kennzeichnung erfolgt das Kennzeichnen mit der ersten Ident-Kennzeichnung nach folgenden Varianten, welche allesamt alternativ und/oder kumulativ zu verstehen sind: Zunächst kann es für die erste Ident-Kennzeichnung, wie bereits für das Verdeckungsmittel ausgeführt, gleichermaßen von Vorteil sein, deren Verbindung zur Verbrauchseinheit in nicht-zerstörungsfrei lösbarer Form auszugestalten, um einen Originalzustand erkennbar zu machen. Die erste Kennzeichnung kann bevorzugt mittels Stanzen, Lasern, Drucken, Prägen, Beschichten, Coating, Inlay-Verfahren, festem Verkleben, 3D-Printing und/oder Versiegeln ausgeführt werden, welche produktionstechnisch vielfältig anpassbare, industriell zuverlässige und effektive Kennzeichnungsverfahren darstellen. Im Sinne eines sinnvollen Layouts der Herstellung der Verbrauchseinheit kann die erste Kennzeichnung in eine Qualitätssicherungsstufe und/oder in eine Verpackungsstufe der Herstellung der Verbrauchseinheit integriert sein. Insbesondere insofern als vielfach Verbrauchsprodukte bereits in-line, quasi als letzten Herstellungsschritt, verpackt werden, kann es in einer Variante bevorzugt sein, auf einer jeweiligen Umverpackung der Verpackungseinheit die erste Kennzeichnung durchzuführen. Diese Variante kann bevorzugt sein, wenn die Umverpackung der Verbrauchseinheit hinzugerechnet werden kann, beispielsweise im Falle einer eine notwendige Sterilität sichernden Umverpackung. Vorzugsweise liegt in einer weiteren alternativen oder kumulativen Variante die erste Ident-Kennzeichnung optisch sichtbar unter einer transparenten jeweiligen Umverpackung der Verpackungseinheit wie einer Klarsichtfolie. Die Verbindung der ersten Kennzeichnung kann als eine zumindest in einem original-verpackten und/oder original-verschlossenen Zustand der Verbrauchseinheit unlösbar ausgeführt sein. Eine so integral ausgestaltete Verbindung bietet den Vorteil, dass anhand der Verbrauchseinheit jeglicher Versuch einer Identitätsentwendung zu unberechtigten Zwecken schwerlich unbemerkt bliebe. Weiter bevorzugt kann die erste Ident-Kennzeichnung in einem zumindest im dem original-verpackten und/oder original-verschlossenen Zustand verdeckten und/oder von außen nicht zugänglichen Bereich der Verbrauchseinheit, wie innenliegend in einer Verschlusskappe, vorgesehen sein. Das dient einer weiteren Behinderung von Möglichkeiten zu Missbrauch oder Manipulation, insofern als bis zum Zeitpunkt eines initialen Anbrechens bzw. bis zum Öffnen eines Verschlusses die erste Ident-Kennzeichnung entzogen ist.

In einer technisch vorteilhaften Ausführungsform des Verfahrens zur Kennzeichnung erfolgt zumindest einer der beiden Schritte Verdecken mit dem Verdeckungsmittel sowie Kennzeichnen mit der zweiten Ident-Kennzeichnung in einer kumulativ und/oder alternativ zu verstehenden Variante: Vorzugsweise sind das auf die erste Ident-Kennzeichnung aufgebrachte Verdeckungsmittel und/oder die auf die Verbrauchseinheit aufgebrachte zweite Ident-Kennzeichnung für die erste Ident-Kennzeichnung zerstörungsfrei lösbar. Bevorzugte Herstellverfahren zur Ausbildung einer lösbaren Verbindung stellen reversible Oberflächenverfahren und/oder Mehrschichtverfahren dar, vorzugsweise mittels Aufbringen einer Aufkleberdeckschicht und/oder einer abrubbelbaren Deckschicht und/oder einer Trennzwischenschicht und/oder auf eine lösbare Haftschicht und/oder mittels Drucken mit reversibler Druckfarbe. Auf diese Weise können robuste Herstellprozesse gefahren werden und die Herstellstufe auf gegebenenfalls vorhandene Maschinen integriert werden. Es kann bevorzugt sein, dass zumindest einer der oben genannten beiden Verfahrensschritte in einer Qualitätssicherungsstufe der Herstellung der Verbrauchseinheit und/oder in einer Verpackungsstufe der Herstellung der Verbrauchseinheit erfolgen. Dies dient einer möglichst integralen Gestaltung des Fabriklayouts und zugehöriger Produktionsabläufe. Ganz besonders kann es dazu bevorzugt sein, dass zumindest einer der Schritte in-line im direkten Anschluss zu und/oder in einer integralen Verfahrensstufe mit dem Schritt Kennzeichnen mit der ersten Ident-Kennzeichnung ausgeführt wird. Vorzugsweise sind das auf die erste Ident-Kennzeichnung aufgebrachte Verdeckungsmittel und/oder die auf die Verbrauchseinheit aufgebrachte zweite Ident-Kennzeichnung auf einer jeweiligen Umverpackung der Verpackungseinheit und/oder optisch sichtbar unter einer transparenten jeweiligen Umverpackung der Verpackungseinheit aufgebracht. Dies erhöht die Freiheitsgrade der Produktgestaltung und der Produktions- bzw. Verpackungsabläufe. Dies kann von besonderem Nutzen sein z.B. in solchen Fällen, wo nationale Erfordernisse besondere und besonders komplexe gesetzliche Vorschriften zur Kennzeichnung von kennzeichnungspflichtigen Verbrauchsprodukten mit Implikationen auf die Verpackungsabläufe vorsehen, beispielsweise in Form von spezifischen Beipackzetteln bzw. Primärverpackungen in der jeweiligen Amtssprache eines jeweiligen Landes. Insbesondere kann die zweite Ident-Kennzeichnung in Form eines zu Logistikzwecken verwendbaren Labels vorgesehen sein. Beispielsweise kann es sich um ein RFID-Label und/oder insbesondere um ein zum Speichern einer Transaktionskette eingerichtetes Label handeln. Im Falle des nicht mit der zweiten Ident-Kennzeichnung zusammenfallenden Verdeckungsmittels kann es bevorzugt sein, dass die zweite Ident-Kennzeichnung nicht auf jeder einzelnen Verbrauchseinheit, sondern nur auf einer Doppel-, Mehrfach-, bzw. Großpackung mit mehreren Verbrauchseinheiten vorgesehen wird. Insofern dient die zweite Ident-Kennzeichnung hauptsächlich zu Versandzwecken und stellt dies eine besonders ressourcenschonende, umweltfreundliche und seitens des Zeitpunkt des Aufbringens der zweiten Ident-Kennzeichnung besonders flexible Variante dar.

Gemäß einem dritten Aspekt der vorliegenden Erfindung wird ein Verfahren zur Freigabe einer Verbrauchsanwendung einer Verbrauchseinheit gemäß dem ersten Aspekt der Erfindung in einer zugehörigen Verbrauchsvorrichtung vorgeschlagen, vorzugsweise in einem Medizinapparat, mit Sicherung gegen Manipulation oder Missbrauch durch unberechtigte Dritte, mit den Schritten eines Entfernens des Verdeckungsmittels von der Verbrauchseinheit an einem Verbrauchsort als Ort eines Verbrauchers und/oder eines Anwenders; und eines Autorisierens des Verbrauches und/oder eines jeweilig zugehörigen Verbrauchszweckes der Verbrauchseinheit. Dabei wird durch das Entfernen des Verdeckungsmittels die erste Ident-Kennzeichnung erkennbar, vorzugsweise optisch sichtbar, freigelegt. Zum Autorisieren wird zumindest der erste Ident-Code erkannt. Über den ersten Ident-Code und die zugehörige erste Datenrelation können die zuvor bei dem Hersteller zugeordnet hinterlegte und gespeicherte Produkttype und/oder Produktverbrauchseigenschaft der Verbrauchseinheit als Information in einem Freigabe-Steuerelement herangezogen werden.

In einer technisch vorteilhaften Ausführungsform des Verfahrens zur Freigabe wird in dem Schritt des Autorisierens weiterhin der zweite Ident-Code erkannt. Über den zweiten Ident-Code und die zugehörige zweite Datenrelation (zum ersten Ident-Code) sind nun auch die für den ersten Ident-Code verfügbaren Informationen, einschließlich der Produkttype und/oder Produktverbrauchseigenschaft bzw. der ersten Datenrelation, verknüpft und über den Hersteller berechtigt beziehbar. Unter Ergänzung der für den ersten Ident-Code vorhandenen Information mit der für den zweiten Ident-Code vorhandenen Information können weitere Rückschlüsse zur Überwachung und Sicherstellung einer berechtigten und/oder passenden Verbrauchsanwendung getroffen werden. Beispielsweise kann die Information eine Lieferkette bzw. Zieladresse eines Verbrauchers bzw. Anwenders zusätzlich einfließen. Das dient einer erhöhten Transparenz und damit Sicherung der Berechtigung.
Vorzugsweise findet die Ident-Code-Erkennung der zweiten Ident-Kennzeichnung, beispielsweise durch einen Scanner, vorab zu der und/oder im selben Zuge mit der Ident-Code-Erkennung der ersten Ident-Kennzeichnung statt. Damit handelt es sich um eine besonders effektive, schnelle Form des Erkennens in einem Vorgang. Andererseits kann es gerade bevorzugt sein, dass die erste und zweite Ident-Kennzeichnung separat erfasst bzw. erkannt bzw. gescanned werden.

In einer technisch vorteilhaften Ausführungsform des Verfahrens zur Freigabe wird zumindest der erste Ident-Code durch den Verbraucher und/oder den Anwender visuell gelesen bzw. mittels eines separaten Code-Lesegeräts erkannt und an die Verbrauchsvorrichtung weitergegeben; und/oder durch die Verbrauchsvorrichtung automatisch erkannt.

In einer technisch vorteilhaften Ausführungsform des Verfahrens zur Freigabe wird aufgrund einer Verbindung des Verdeckungsmittels, insbesondere der zweiten Ident-Kennzeichnung, mit der Verbrauchseinheit das Entfernen des Verdeckungsmittels im Zuge eines Verbrauchs der Verbrauchseinheit mitbewirkt, vorzugsweise im Zuge des Öffnens der Verbrauchseinheit, bspw. beim Abnehmen eines Deckels oder einer Verschlusskappe, und/oder eines Anschließens der Verbrauchseinheit an die Verbrauchsvorrichtung. Das dient besonders zügigen und konvenienten Abläufen bei der Verbrauchsanwendung, z.B. im klinischen Alltag.

In einer technisch vorteilhaften Ausführungsform des Verfahrens zur Freigabe ist als weiterer Schritt eine Sperrwirkung der Verbrauchseinheit für einen Verbrauch vorgesehen. So kann eine Sperrwirkung nach einem Ablehnen bzw. Fehlschlagen des Autorisierens ausgelöst werden, wobei die Nicht-Einhaltung einer zum Autorisieren zu erfüllenden Bedingung unter anderem folgende auslösende Gründe kumulativ und/oder alternativ betreffen kann: einen für den Verbraucher und/oder den Anwender zuvor hinterlegten zulässigen Verbrauchszweck wie beispielsweise eine ärztliche Wiederholungsverschreibung; auf Basis einer Überprüfung der Produkttype und/oder Produktverbrauchseigenschaft der Verbrauchseinheit; eine der Verbrauchseinheit zugeordnete Zeitobergrenze, vorzugsweise auf Basis einer Haltbarkeitsdauer und/oder eines Zeitfensters ab Erreichen des Verbrauchsorts und/oder ab Erkennen des ersten Ident-Codes; und/oder eine Kompatibilität von technischen Anforderungen zum ordnungsgemäßen Verbrauch der Verbrauchseinheit mit den technischen Daten der Verbrauchsvorrichtung. Dabei kann im Falle einer Sperrwirkung vorzugsweise weiterhin noch eine Information über einen auslösenden Grund und/oder zu einer Problemlösung durch den Hersteller erfolgen.

In einer technisch vorteilhaften Ausführungsform wird ein Verfahren zur Systemsteuerung zur Ausführung einer Ausführungsform des zuvor beschriebenen Verfahrens zur Freigabe in Kombination mit einer Ausführungsform des zuvor beschriebenen Verfahrens zur Kennzeichnung vorgeschlagen. Dabei erfolgt der Schritt des Autorisierens mittels informationstechnischem Abgleichen des an dem Verbrauchsort erkannten ersten Ident-Codes mit dem generierten und durch den Hersteller gespeicherten ersten Ident-Code. Vorzugsweise kann das Abgleichen in kumulativ und/oder alternativ zu verstehenden Ausführungsformen erfolgen: mit einer in der Verbrauchsvorrichtung zuvor hinterlegten Kopie des generierten ersten Ident-Codes; mit einer bei dem Verbraucher und/oder dem Anwender zuvor hinterlegten Kopie des generierten ersten Ident-Codes; mit einer bei einer dezentralen Datenbank eines berechtigten weiteren Teilnehmers, beispielsweise einer computer-basierten Instanz einer Krankenkasse, zuvor hinterlegten Kopie des generierten ersten Ident-Codes. Vorzugsweise können das Abgleichen und/oder das Übermitteln der zuvor hinterlegten Kopie mittels Fernkommunikation über eine Schnittstelle zu einer Datenbank des Herstellers zur Verwaltung zumindest des ersten Ident-Codes erfolgen.

In einer besonders bevorzugten Ausführungsform des vorangehend beschriebenen Verfahrens zur Systemsteuerung werden hinsichtlich des Verfahrens zur Kennzeichnung die erste und die zweite Ident-Kennzeichnung sowohl schichtweise als auch farblich codiert. Herstellerseitig wird in der Produktion dabei nur ein Teil des Farbspektrums und/oder die additive Farbmischung als ein Mischcode erfasst. Später am Verbrauchsort werden hinsichtlich des Verfahrens zur Freigabe die die erste und die zweite Ident-Kennzeichnung ausbildenden Schichten zunächst getrennt und dann sowohl der Mischcode als auch die erste und die zweite Ident-Kennzeichnung jeweils einzeln gescannt. Ist die obere Schicht in Form der ersten Ident-Kennzeichnung einmal entfernt, ist eine Repositionierung wieder auf die ursprüngliche Position nicht mehr exakt möglich. Somit besteht eine vorteilhafte Möglichkeit, durch einen Vergleich vom Scan des Mischcodes und der Code-Berechnung von der erste und der zweiten Ident-Kennzeichnung die Verbrauchseinheit auf eine etwaig stattgefundene Manipulation prüfen.

In einer technisch vorteilhaften Ausführungsform des Verfahrens zur Systemsteuerung umfasst der Schritt des Autorisierens als weiteren Schritt eine informationstechnische Verwendung der zweiten Datenrelation zur Berücksichtigung einer bis zum Verbrauchsort zu Herstellprozess und Logistik gesammelten Information und/oder Transaktionskette.

In einer technisch vorteilhaften Ausführungsform des Verfahrens zur Systemsteuerung umfasst der Schritt des Autorisierens als weiteren Schritt eine informationstechnische Verwendung der ersten Datenrelation zur Berücksichtigung der Produkttype und/oder Produktverbrauchseigenschaft der Verbrauchseinheit.

In einer technisch vorteilhaften Ausführungsform des Verfahrens zur Systemsteuerung wird ein informationstechnischer Schritt in zumindest einer Transaktionskette, vorzugsweise in einer Blockkette mit verknüpften Transaktionen in einem zur Gewährleistung von Dokumentationssicherheit zwischen mehreren verteilten Teilnehmern eingerichteten Datenbankmanagement-Steuersystem, verarbeitet und/oder hinterlegt. Insbesondere betrifft dies einen oder mehrere der Schritte: Code-Generieren; Speichern durch den Hersteller, vorzugsweise von Daten aus Produktion und/oder Logistik; Zuordnen zur Bildung der ersten Datenrelation, Zuordnen zur Bildung der zweiten Datenrelation; Autorisieren; Abgleichen; Überprüfen; Verwendung der ersten Datenrelation, Verwendung der zweiten Datenrelation; Sperrwirkung; und/oder Information. Sofern ein Datenbankmanagement-Steuersystem vorgesehen ist, ist dieses vorzugsweise eingerichtet, das Verfahren insbesondere zum Zwecke der Dokumentation einer Akte eines Teilnehmers, wie einer Patientenakte, und/oder zum Zwecke der Versandplanung an Verbrauchsorte, wie Einrichtungen für medizinische Anwendungen und/oder zum Zwecke einer Überwachung auszuführen.

Der Begriff "Transaktionskette" bzw. eine sogenannte "Blockchain" (englisch für: "Blockkette") meint allgemein eine kontinuierlich erweiterbare Liste von Blöcken in Form von zu speichernden Datensätzen. Der Inhalt der Blöcke repräsentiert insbesondere initiale, historische, zeitliche und/oder örtliche Stammdaten und Verwendungsdaten des Verbrauchsgegenstandes. Dabei können die Blöcke mittels kryptographischer Verfahren verschlüsselt sein. Zudem können die Blöcke miteinander verkettet sein, um eine Kette von Blöcken zu bilden. Insbesondere kann ein Block einen kryptographisch sicheren "Hash"wert (englisch für: Streuwert) des vorhergehenden Blocks, beispielsweise einen Zeitstempel, eine Signatur und/oder Transaktionsdaten, enthalten. Durch eine aufeinander aufbauende Speicherung der Blöcke können diese nicht unbemerkt nachträglich geändert werden, was die Integrität des Gesamtsystems bzw. der Blockchain sicherstellt. Hierdurch wird einer lokalen Datenmanipulation der Blöcke durch dezentrale Kontrolle entgegengewirkt. Mit anderen Worten wird Dokumentierungssicherheit im zeitlichen Verlauf bzw. entlang der Datenhistorie erzielt, insbesondere was Datenversionen der Blöcke betrifft.

Insbesondere umfasst der Begriff "Transaktionskette" auch ein verteiltes Datenbankmanagementsystem mit mehreren Teilnehmern, vorzugsweise ein dezentral geführtes dokumentierungssicheres Datendokumentationssystem, beispielsweise im Bereich medizinischer Anwendungen und Therapien, wie z.B. einer Dialyse.

Unter den Begriff des "Teilnehmers" fallen computer-basierte Instanzen, die unterschiedlichen Entitäten zugeordnet sein können, nämlich: dem Hersteller des Verbrauchsproduktes einschließlich dessen Rohstoff- und Komponentenzulieferern; dem Verbraucher als Endkonsumenten z.B. einem Patienten; dem Anwender, vorzugsweise einem klinischen oder therapeutischen Anwender, wie einem Arzt, einer Krankenschwester, einem Pflegeroboter; einer gesundheitlichen Koordinierungsstelle; einem logistischen Dienstleister, wie einem Transportunternehmen etc.

Der Begriff eines "(verteilten) Datenbankmanagement-Steuersystems" bezieht sich auf einen im allgemeinen sogenannten "Distributed Ledger" (engl. für: "verteiltes Geschäftsbuch"; häufig fachsprachlich abgekürzt als: DLT, Technology), also auf ein dezentrales Speichersystem. Insbesondere ist der verteilte Ledger in dem Sinne verteilt, dass mehrere Kopien von Blöcken des verteilten Ledger in verschiedenen Speichereinheiten (mitunter auch räumlich) verteilt sind. Der verteilte Ledger umfasst mehrere auch einzeln adressierbare Datensätze. Insbesondere sind die mehreren Datensätze als Datenblöcke organisiert. Insbesondere werden die Blöcke durch unterschiedliche Instanzen, insbesondere verschiedene computer-basierte Knoten eines Netzwerks, erstellt und auf den verschiedenen Instanzen gespeichert. Mit anderen Worten erfolgen der Aufbau und die Wartung der Datensätze üblicherweise nicht von einer zentralen Instanz. In typischen Fällen behalten alle, zumindest aber eine sehr große Anzahl von Knoten des Netzwerks eine Kopie des verteilten Ledgers. Im Allgemeinen basiert die Speicherung und die Aktualisierung des verteilten Ledgers und die Abfrage von Daten aus dem Ledger typischerweise auf einem Konsensmechanismus. Der Konsensmechanismus sorgt dafür, dass die verschiedenen Kopien des verteilten Ledgers übereinstimmen, so dass gesamthaft Manipulationsmöglichkeiten und/oder Fehlerquellen strukturell-technisch vorgebeugt ist und eine Dokumentenechtheit und auch eine Dokumentensicherheit der zeitlich veränderlichen Versionen der Datensätze technisch unterstützt bzw. gewährleistet ist.

Gemäß einem vierten Aspekt der vorliegenden Erfindung wird ein Steuersystem vorgeschlagen, welches zur Ausführung eines oben beschriebenen Verfahrens zur Systemsteuerung eingerichtet ist. Dabei umfasst das Steuersystem: ein Steuergerät sowie eine Kennzeichnungsvorrichtung, eingerichtet zum gleichzeitigen und/oder separaten Aufbringen zumindest der ersten Ident-Kennzeichnung und/oder des Verdeckungsmittels an dem Ort des Herstellers; und/oder die Verbrauchsvorrichtung, zum Verbrauch des Verbrauchsprodukts an dem Ort des Verbrauchers und/oder des Anwenders.
Weiterhin kann es in einer bevorzugten Ausführungsform das Steuersystem ein zwischen mehreren verteilten Teilnehmern eingerichtetes Datenbankmanagement-Steuersystem umfassen, welches vorzugsweise als Blockkette mit verknüpften Transaktionen zur Gewährleistung von Dokumentationssicherheit ausgestaltet ist.

In einer technisch vorteilhaften Ausführungsform wird ein Computerprogramm vorgesehen, welches, geladen in einen digitalen Speicher eines Computers, eine Ausführungsform der oben beschriebenen Verfahren zur Kennzeichnung und/oder zur Freigabe gemäß dem zweiten bzw. dritten Aspekt der Erfindung implementiert und/oder ein Steuersystem gemäß dem vierten Aspekt der Erfindung ausführt.

Gemäß einem fünften Aspekt der vorliegenden Erfindung wird ein Sicherheitselement vorgeschlagen, welches zum Aufbringen auf eine Verbrauchseinheit für eine Verbrauchsanwendung eines Verbrauchsprodukts, vorzugsweise zum Verbrauch in einem Medizinapparat, ausgestaltet und zur Sicherung der Verbrauchseinheit gegen Manipulation oder Missbrauch durch unberechtigte Dritte eingerichtet ist. Das erfindungsgemäße Sicherheitselement umfasst: eine Kombination aus einer einen ersten Ident-Code materiell ausbildenden ersten Ident-Kennzeichnung und einer einen zweiten Ident-Code materiell ausbildenden zweiten Ident-Kennzeichnung. Dabei ist die erste Ident-Kennzeichnung eingerichtet, eine unlösbare Verbindung zu der Verbrauchseinheit auszubilden und eine eindeutige Identifizierung der Verbrauchseinheit, wie hinsichtlich einer Produkttype und/oder Produktverbrauchseigenschaft der Verbrauchseinheit, zu stellen. Weiter ist die erste Ident-Kennzeichnung mit einem, vorzugsweise nicht-zerstörungsfrei lösbaren Verdeckungsmittel teilweise oder vollständig verdeckt, vorzugsweise optisch verdeckt. Ferner ist die zweite Ident-Kennzeichnung lösbar und zu einer vorübergehenden Verwendung, etwa zum Zwecke der logistischen Nachverfolgbarkeit, eingerichtet. Vorzugsweise ist die zweite Ident-Kennzeichnung identisch zu dem Verdeckungsmittel. Alternativ oder kumulativ ist das Verdeckungsmittel nicht-zerstörungsfrei lösbar.

In der folgenden detaillierten Figurenbeschreibung werden nicht einschränkend zu verstehende Ausführungsbeispiele mit deren Merkmalen und weiteren Vorteilen anhand der Zeichnung besprochen. Es zeigen:
- Fig. 1: eine Seitenansicht einer mit einer ersten Ident-Kennzeichnung gekennzeichneten Verbrauchseinheit;
- Fig. 2: eine Seitenansicht einer erfindungsgemäßen Verbrauchseinheit;
- Fig. 3: eine Seitenansicht einer Verbrauchseinheit gemäß einem bevorzugten Ausführungsbeispiel der vorliegenden Erfindung;
- Fig .4: ein Flussdiagramm veranschaulichend Schritte eines Ausführungsbeispiels eines erfindungsgemäßen Verfahrens zur Kennzeichnung;
- Fig. 5: ein Flussdiagramm veranschaulichend Schritte eines Ausführungsbeispiels eines erfindungsgemäßen Verfahrens zur Freigabe;
- Fig. 6: eine schematische Darstellung eines Steuersystems gemäß einem Ausführungsbeispiel der vorliegenden Erfindung mit einer Verbrauchseinheit gemäß dem bevorzugten Ausführungsbeispiel der vorliegenden Erfindung;
- Fig. 7: eine Seitenansicht eines Verbrauchsproduktes in einer Sekundärverpackung mit mehreren Verbrauchseinheiten gemäß einem weiteren bevorzugten Ausführungsbeispiel der vorliegenden Erfindung.

### Detaillierte Beschreibung von Ausführungsbeispielen

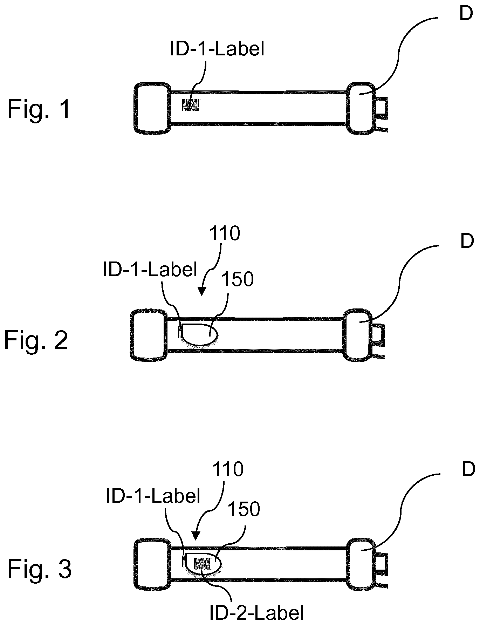

Figuren 1 und 2 zeigen jeweils eine Seitenansicht eines Verbrauchsgegenstandes zur Verwendung im Rahmen eines medizintechnischen Gerätes, beispielsweise in Form eines Dialysemoduls D (Dialysator, Schlauchset, etc.), das für einen Patienten an einem Dialysegerät zu verwenden ist. Das Dialysemodul D ist mit einem mehrzeiligen Barcode-Aufdruck ID-1-Label als einer ersten Ident-Kennzeichnung mit Permanentdruckfarbe bedruckt, sowie eine Seitenansicht eines erfindungsgemäßen Dialysemoduls D mit einem abziehbar aufgeklebten Sicherheitselement 110. Die graphische Darstellung des mehrzeiligen Barcode-Aufdrucks ID-1-Label findet auf informationstechnischer Seite eine Entsprechung in einem (nicht abgebildeten) herstellerseitigen Steuersystem 400, in welchem der zugehörige numerische Datencode ID-1-Code generiert und unter einer fortlaufenden Seriennummer als eine Stammdateninformation Type mitabgelegt wird. Wie in Fig. 2 erkennbar, ist der mehrzeilige Barcode ID-1-Label mit Ausnahme seiner ersten Zeile mit einem abziehbaren neutral gestalteten Aufkleber 150 mit einer eckig ausgeformten Abziehlasche optisch verdeckt, wodurch das Sicherheitselement 110 ausgebildet ist. Das Sicherheitselement schützt den abgeklebten Teil des Barcodes ID-1-Label gegen ein Auslesen bzw. Scannen. Die erste unverdeckte Zeile kann wie ein einzeiliger Barcode z.B. in der Qualitätssicherung und zum Versand herkömmlich ausgelesen und verwendet werden. Sobald der Aufkleber 150 entfernt ist, entspricht der Zustand des Dialyse-Moduls wieder dem vor dem Verdecken, wie in Fig. 1 gezeigt. Dann kann der vollständige Barcode ID-1-Label, also alle seine Zeilen, in einem Barcode-Scanner ausgelesen und zu einer Identifikation des individuellen Dialysemoduls D herangezogen werden.

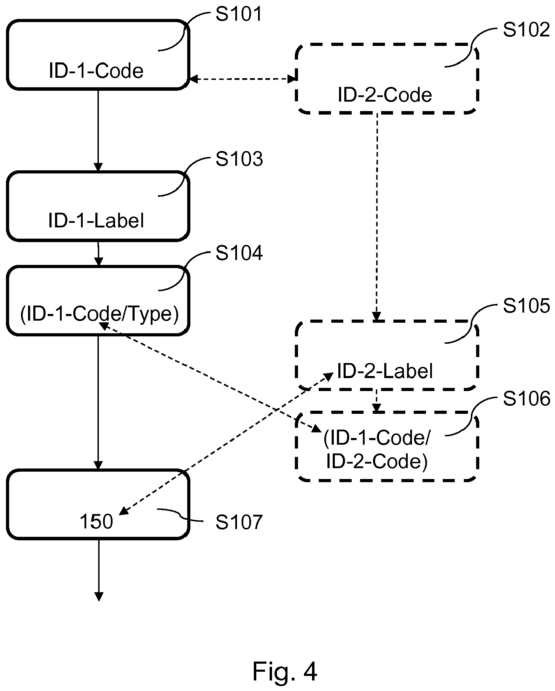

Die beiden Figuren 1 und 2 veranschaulichen gleichermaßen zwei Herstellstufen eines erfindungsgemäßen Verfahrens zur Kennzeichnung, wie es schematisch anhand von Figur 4 unten beschrieben wird, wobei Fig. 1 eine halbfertige Zwischenstufe und Fig. 2 ein fertiges erfindungsgemäßes Dialysemodul D mit einem Sicherheitselement 110 zeigt.

Fig. 3 zeigt eine Seitenansicht eines Dialysemoduls D gemäß einem bevorzugten Ausführungsbeispiel der vorliegenden Erfindung. Dabei enthält der in Fig. 2 dargestellte Aufkleber 150 einen zweiten mehrzeiligen Barcode-Aufdruck ID-2-Label als eine zweite Ident-Kennzeichnung. Der zweite mehrzeilige Barcode-Aufdruck ID-2-Label ist optisch gut sichtbar außen an dem Dialysemodul D und kann somit von jedermann eingescanned und genutzt werden.

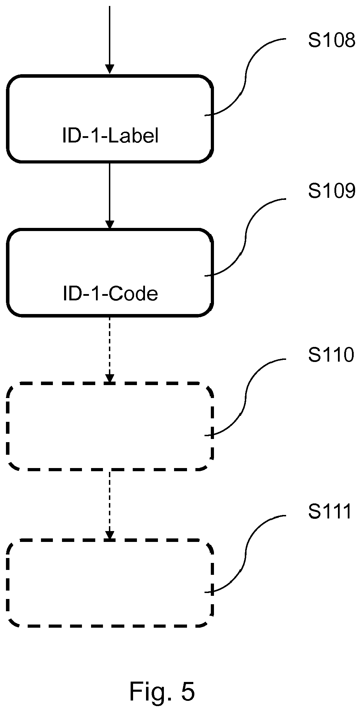

Fig. 4 und Fig. 5 zeigen jeweils ein Flussdiagramm, welches Schritte S101-S107 eines Ausführungsbeispiels eines erfindungsgemäßen Verfahrens zur Kennzeichnung einer (nicht dargestellten) Verbrauchseinheit für eine Verbrauchsanwendung eines Verbrauchsprodukts (Fig. 4) beziehungsweise Schritte S108-S111 eines Verfahrens zu dessen Freigabe (Fig. 5) veranschaulicht. Beide Verfahren dienen einer Absicherung einer, einer einzelnen Verbrauchseinheit in Form einer ersten Ident-Kennzeichnung ID-1-Label verliehenen, Identität gegen Manipulation oder Missbrauch durch unberechtigte Dritte. Durch das in Fig. 4 veranschaulichte Verfahren wird zunächst die Verbrauchseinheit mit einem herstellerseitigen Sicherheitselement an einem Herstellerort gesichert. Und nach einem Transport der Verbrauchseinheit an einen Verbrauchsort wird das in Fig.5 veranschaulichte Verfahren zu deren Freigabe für einen ordnungsgemäßen wie passenden Verbrauch für einen Berechtigten ausgeführt. Dabei sei hinsichtlich der schematischen Darstellungsweise darauf hingewiesen, dass die für die vorliegende Erfindung wesentlichen Schritte in jeweiligen Kästchen mit durchgezogen-linierter Umrandung repräsentiert sind (links in Fig. 4: S101, S103, S104 und S107; oben in Fig. 5: S108 und S109). Hingegen sind die lediglich optionalen Schritte mit gestrichelt-linierter Umkastelung dargestellt, insofern als sie für bevorzugte Ausführungsformen der Erfindung anfallen (rechts in Fig. 4: S102, S105 und S106; unten in Fig. 5: S110 und S111).

Das in Fig. 4 mit dem Flussdiagramm schematisierte Verfahren zur Kennzeichnung einer Verbrauchseinheit umfasst erfindungswesentlich die Schritte S101, S103, S104 und S107: In einem ersten Schritt S101 wird ein erster Ident-Code ID-1-Code informationstechnisch generiert bzw. rechentechnisch erzeugt und durch einen Hersteller des Verbrauchsproduktes gespeichert. In einem nachfolgenden Schritt S103 wird die Verbrauchseinheit mit einer ersten Ident-Kennzeichnung ID-1-Label gekennzeichnet, welche den ersten Ident-Code ID-1-Code materiell ausbildet. Parallel zu Schritt S103 erfolgt auf informationstechnischer Ebene ein Zuordnen S104 von einer Produkttype Type und/oder Produktverbrauchseigenschaft der Verbrauchseinheit zu dem ersten Ident-Code ID-1-Code, wodurch eine erste Datenrelation ID-1-Code/Type gebildet wird. Der Schritt S104 bzw. die erste Datenrelation ID-1-Code/Type wird durch den Hersteller gespeichert. In einem weiteren Schritt S107 wird die erste Ident-Kennzeichnung ID-1-Label mit einem Verdeckungsmittel 150 verdeckt. Das Verdecken S107 erfolgt teilweise oder vollständig, vorzugsweise optisch, und mittels eines von der ersten Ident-Kennzeichnung ID-1-Label (wieder) lösbaren Verdeckungsmittel 150.

Weiterhin kann das Verfahren zur Kennzeichnung optionale Schritte umfassen: informationstechnisches Generieren S102 eines zweiten Ident-Codes ID-2-Code und Speichern durch den Hersteller; Kennzeichnen S105 der Verbrauchseinheit mit einer nicht-verdeckten zweiten Ident-Kennzeichnung ID-2-Label; und informationstechnisches Zuordnen S106 des zweiten Ident-Codes ID-2-Code zu dem ersten Ident-Code ID-1-Code zur Bildung einer zweiten Datenrelation ID-1-Code/ID-2-Code und Speichern durch den Hersteller. Anhand des gestrichelt-linierten Doppelpfeils zwischen Schritt S104 und Schritt S106 ist veranschaulicht, dass über den ersten Ident-Code ID-1-Code die erste Datenrelation ID-1-Code/Type und die zweite Datenrelation ID-1-Code/ID-2-Code verknüpfbar sind, womit indirekt der zweite Ident-Code ID-2-Code und die Produkttype Type und/oder Produktverbrauchseigenschaft zueinander informationstechnisch bezogen sind. Anhand des gestrichelt-linierten Doppelpfeils zwischen Schritt S107 und Schritt S105 wiederum ist veranschaulicht, dass in einer bevorzugten Ausführungsform das Verdeckungsmittel 150 und die zweite Ident-Kennzeichnung ID-2-Label identisch sind bzw. zusammenfallen.

Das in Fig. 5 mit dem Flussdiagramm schematisierte Verfahren zur Freigabe einer Verbrauchseinheit umfasst erfindungswesentlich die Schritte S108 und S109: eines Entfernens S108 des Verdeckungsmittels von der Verbrauchseinheit an einem Verbrauchsort und eines Autorisierens S109 des Verbrauches und/oder eines jeweilig zugehörigen Verbrauchszweckes der Verbrauchseinheit. Im Schritt Entfernen S108 wird die erste Ident-Kennzeichnung ID-1-Label durch Entfernen des Verdeckungsmittels (wieder) erkennbar, vorzugsweise optisch sichtbar, freigelegt. Das Entfernen kann z.B. im Rahmen des Öffnens einer Verschlusskappe ausgebildet sein. Zum Autorisieren S109 wird zumindest der erste Ident-Code ID-1-Code erkannt. Über den ersten Ident-Code und die zugehörige erste Datenrelation können die zuvor bei dem Hersteller zugeordnet hinterlegte und gespeicherte Produkttype und/oder Produktverbrauchseigenschaft der Verbrauchseinheit als Information in einem Freigabe-Steuerelement herangezogen werden. Weiterhin kann das Verfahren zur Freigabe optional die Schritte Auslösen bzw. Initiieren einer Sperrwirkung S110 und/oder Ausgabe von Information S111 (z.B. über eine unberechtigterweise intendierte Verwendung) umfassen.

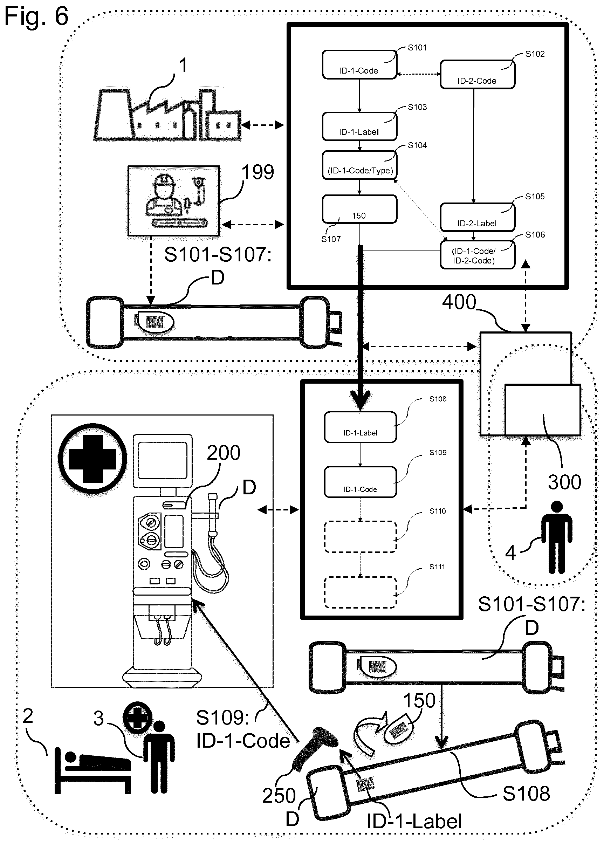

Fig. 6 zeigt eine schematische Darstellung eines Steuersystems 400 (mit einem nicht dargestellten Speicher) gemäß einem Ausführungsbeispiel der vorliegenden Erfindung zum missbrauchssicheren Verbrauch einer bevorzugten Ausführungsform des Dialysemoduls D aus Fig. 3 in einem zugehörigen Dialysegerät 200 als einer zugehörigen Verbrauchsvorrichtung, d.h. für eine präskriptionsgemäße Dialysebehandlung eines Patienten 2 unter Aufsicht eines Arztes 3. Das Steuersystem führt dazu eine bevorzugte Ausführungsform des Verfahrens zur Kennzeichnung (vergleiche Fig. 4) von Dialysemodulen D in Kombination mit der nachfolgenden Ausführung einer bevorzugten Ausführungsform des Verfahrens zur Freigabe (vergleiche Fig. 5) der Dialysemodule D zu einem jeweils berechtigten Verbrauch aus. Das Verfahren zur Kennzeichnung einerseits wird in einem oben in der Fig. 6 befindlichen Einflussbereich eines Herstellers 1 von Dialysemodulen D mit einer Kennzeichnungsvorrichtung 199 ausgeführt. Das Verfahren zur Freigabe andererseits wird in einem unten in der Fig. 6 befindlichen Einflussbereich des Patienten 2 bzw. des Arztes 3 ausgeführt.

Ferner ist eine computer-basierte Instanz einer gesundheitlichen Koordinierungsstelle 4 als ein weiterer, dezentraler Teilnehmer mittig rechts in Fig. 4 schematisch dargestellt, die ausgebildet ist, ein verteiltes Datenbankmanagement-Steuersystem 300 (mit einem nicht dargestellten Speicher) zu betreiben. Das Datenbankmanagement-Steuersystem 300 ist insbesondere zwischen den computer-basierten Instanzen der Teilnehmer (Hersteller 1, Patient 2, Anwender 3 und Koordinierungsstelle 4) verteilt. Das Datenbankmanagement-Steuersystem 300 stellt einen Teil des Steuersystems 400 dar, mit welchem es einen überschneidenden Bereich ausbildet. Die vier lokal verteilten Einflussbereiche der verteilten Teilnehmer 1, 2, 3 und 4 sind graphisch in Form von gestrichelt-linierten Domänen bzw. Untereinheiten angedeutet. Gestrichelt-Iinierte Pfeile zum Steuersystem 400 mit Datenbankmanagement-Steuersystem 300 der Koordinierungsstelle 4 symbolisieren die jeweilige Teilnahme der anderen drei verteilten Teilnehmer 1, 2 und 3.

Die beiden oben genannten Verfahren, einerseits das Verfahren zur Kennzeichnung mit den Verfahrensschritten S101-S107 (vergleiche Fig. 4) sowie andererseits das Verfahren zur Freigabe mit den Verfahrensschritten S108-S111 (vergleiche Fig. 5), sind in fett-liniert umrandeten Verfahrensblockschema-Kästchen in ihren jeweils zugehörigen Einflussbereichen dargestellt, und zwar einerseits in dem Einflussbereich des Herstellers 1 und andererseits in dem Einflussbereich des Patienten 2 und/oder Arztes 3. Die beiden zuvor genannten Verfahrensblockschema-Kästchen sind von oben nach unten durch einen fett-liniert eingetragenen Übergangspfeil verbunden. Der Übergangspfeil symbolisiert die Kombination der beiden oben genannten Verfahren zu einem übergeordneten Verfahren zur Systemsteuerung. Bei dem Verfahren zur Systemsteuerung folgt das Verfahren zur Freigabe auf das Verfahren zur Kennzeichnung. Insofern ist der erste Verfahrensschritt des Verfahrens zur Freigabe S108 (unten) nachfolgend zu dem letzten Verfahrensschritt der Kennzeichnung zur Freigabe. Mit anderen Worten geht eine Ausgabe aus dem Verfahren zur Kennzeichnung in Form des fertigen Dialysemoduls D als einer mit einem Sicherheitselement gekennzeichneten Verbrauchseinheit als eine Eingabe in das Verfahren zur Freigabe ein. Der letzte Verfahrensschritt der Kennzeichnung zur Freigabe entspricht in der in Fig. 6 veranschaulichten Ausführungsform den optionalen Verfahrensschritten S105 mit S106 zur zweiten Ident-Kennzeichnung ID-2-Label mit Code ID-2-Code. Nach einem dazwischenliegenden Transport des fertigen Dialysemoduls D vom Hersteller 1 zu dem Patienten 2 oder Arzt 3 folgt der Schritt S108, welcher einer Sichtbarmachung bzw. einem 'Entdecken' der ersten Ident-Kennzeichnung ID-1-Label dient. Dieser Schritt S108 ist unten rechts in Fig. 6 graphisch detaillierter veranschaulicht. Wie zu sehen, zieht der Arzt 3 den mit dem zweiten mehrzeiligen Barcode-Aufdruck ID-2-Label versehenen Aufkleber 150 von dem Dialysemodul D an der Abziehlasche ab. Dadurch ist nun der erste mehrzeilige Barcode-Aufdruck ID-1-Label (im linken Bereich des zylindrischen Mittelteils des Dialysemoduls D) unbeschadet und wieder vollständig sichtbar freigelegt. Als nächstes scannt der Arzt mit einem Handscanner 250 den ersten mehrzeilige Barcode-Aufdruck ID-1-Label ein, womit eine informationstechnische Übermittlung des zugehörigen Ident-Codes ID-1-Code an das mit dem Handscanner 250 (über eine Kabelverbindung oder drahtlos) kommunizierende verbundene Dialysegerät 200 erfolgt. Sofern anhand des Ident-Codes ID-1-Code positiv festgestellt werden kann (Steuersystem 400, Dialysegerät 200, Schritt Autorisieren S109), dass das vom Arzt 3 ausgelesene Dialysemodul D eine für den Patienten 2 verschriebene bzw. passende Dialysebehandlung durchführen kann und dass ferner das in dem betreffenden Behandlungszimmer aufgestellte Dialysegerät 200 von seiner Gerätebauweise bzw. Modelltype für einen Dialyse-Betrieb mit dem Dialysemodul D als Gerätesystem harmoniert bzw. geeignet konfiguriert ist, wird die Dialysebehandlung autorisiert begonnen. In dem anderen Fall einer negativen Feststellung kann in Schritt S111 optional auf dem Bildschirm des Dialysegerätes eine Information zu möglichen Problemlösungen ausgegeben werden; beispielsweise ein Hinweis an den Arzt 3, dass sich ein zu dem ausgelesenen Dialysemodul D passendes weiteres Dialysegerät in einem anderen Behandlungszimmer befindet oder dass die Haltbarkeitsdauer des Dialysemoduls D überschritten ist, usw. Bei positiver Feststellung bzw. nach erfolgter Dialyse-Behandlung kann eine solche Information bzw. Transaktion an das Steuersystem 400 bzw. das Datenbankmanagement-Steuersystem 300 zur Auslösung weiterer optionaler Funktionalitäten wie weitergegeben werden.

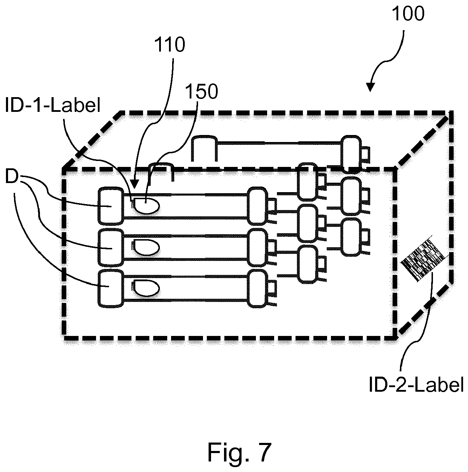

Fig. 7 zeigt eine Seitenansicht eines Verbrauchsproduktes 100 in einem quaderförmigen Versandkarton (Umrisse gestrichelt-liniert angedeutet) als einer Sekundärverpackung mit neun Dialysemodulen D als Verbrauchseinheiten gemäß einem weiteren bevorzugten Ausführungsbeispiel der vorliegenden Erfindung. Jedes der neun Dialysemodule D entspricht der in Fig. 2 dargestellten erfindungsgemäßen Ausführungsform und umfasst insofern ein Sicherheitselement 110 mit einem von einem Aufkleber 150 bis auf die erste Zeile abgeklebten mehrzeiligen Barcode-Aufdruck ID-1-Label. Außen rechts an der Stirnfläche des Versandkartons ist unabhängig, z.B. von einer Spedition, ein sogenanntes "Track-and-Trace-Label" ID-2-Label als zweite Ident-Kennzeichnung zu Versandzwecken wie Nachverfolgbarkeit angebracht. Nach Ankunft an der Zieladresse, beispielsweise einem Dialysezentrum oder einem Dialysepatienten mit wiederkehrendem Gebrauch, werden die Dialysemodule D einzeln dem Versandkarton entnommen und ihrer bestimmungsgemäßen Verwendung zugeführt.

Abschließend sei darauf hingewiesen, dass die Beschreibung der Erfindung und die Ausführungsbeispiele grundsätzlich nicht einschränkend in Hinblick auf eine bestimmte physikalische Realisierung der Erfindung zu verstehen sind. Alle in Verbindung mit einzelnen Ausführungsformen der Erfindung erläuterten und gezeigten Merkmale können in unterschiedlicher Kombination in dem erfindungsgemäßen Gegenstand vorgesehen sein, um gleichzeitig deren vorteilhafte Wirkungen zu realisieren.

Alle Verfahrensschritte können durch Vorrichtungen implementiert werden, die zum Ausführen des jeweiligen Verfahrensschrittes geeignet sind. Alle Funktionen, die von gegenständlichen Merkmalen ausgeführt werden, können ein Verfahrensschritt eines Verfahrens sein. Des Weiteren können die Bauteile bzw. Mittel der der Verbrauchseinheit zugehörigen Verbrauchsvorrichtung, vorzugsweise dem Medizinapparat, sowie des Steuersystems zur Ausführung eines Verfahrens auf mehrere physikalische Produkte bzw. Unterbaugruppen verteilt realisiert werden kann.

Der Schutzbereich der vorliegenden Erfindung ist durch die Ansprüche gegeben und wird durch die in der Beschreibung erläuterten oder den Figuren gezeigten Merkmale nicht beschränkt.

Für einen Fachmann ist es offensichtlich, dass die Erfindung nicht nur für die Sicherung von Dialyseverfahren, also von medizinischen Verbrauchsanwendungen in zugehörigen Dialysegeräten gegen Missbrauch und zur Absicherung gegen eine unberechtigte Nutzung angewendet werden kann, sondern auch für die Absicherung anderer technischer Systemvorrichtungen, in welchen passende Verbrauchseinheiten verbraucht werden. Weitere Einsatzbeispiele der vorgeschlagenen Sicherung und Überwachung gegen Manipulation oder Missbrauch durch unberechtigte Dritte und vorzugsweise einem Auditing bei Systemgeräten für verbrauchsrelevante Informationen (z.B. elektronische Gesundheitsakte) betreffen den Verbrauch von Membranen für Umkehrosmoseanlagen. Es ist ein Kerngedanke der Erfindung hinsichtlich der Verbrauchseinheiten herstellerseitig eine effektive, spezifisch wirksame wie auch vielfältig und flexibel einsetzbare technische Möglichkeit im Sinne der Sicherung der Verbraucher bzw. Anwender und gegebenenfalls der Allgemeinheit gegen Folgen etwaigen diversen Missbrauchs und/oder von Manipulation zu schaffen und damit weiter vorbeugend zu wirken.

### BEZUGSZEICHEN

- 1: Hersteller
- 2: Verbraucher
- 3: Anwender
- 4: weiterer Teilnehmer
- 100: Verbrauchsprodukt
- 110: Sicherheitselement
- 150: Verdeckungsmittel
- 199: Kennzeichnungsvorrichtung
- 200: Verbrauchsvorrichtung (insb. Medizinapparat)
- 250: Code-Lesegerät
- 300: Datenbankmanagement-Steuersystem
- 400: Steuersystem

- D: Verbrauchseinheit (insb. Dialysemodul)
- ID-1-Code: erster Ident-Code
- ID-1-Label: erste Ident-Kennzeichnung
- ID-2-Code: zweiter Ident-Code
- ID-2-Label: zweite Ident-Kennzeichnung
- Type: Produkt-Type/ -Verbrauchseigenschaft
- ID-1-Code/Type: erste Datenrelation
- ID-1-Code /ID-2-Code: zweite Datenrelation
- S101 - S113: Verfahrensschritte

## Patentansprüche

1. Verbrauchseinheit (D) für eine Verbrauchsanwendung eines Verbrauchsprodukts (100), vorzugsweise zum Verbrauch in einem Medizinapparat (200), mit einem Sicherheitselement (110) zur Sicherung gegen Manipulation oder Missbrauch durch unberechtigte Dritte, wobei:
- das Sicherheitselement (110) eine einen ersten Ident-Code (ID-1-Code) materiell ausbildende, erste Ident-Kennzeichnung (ID-1-Label) umfasst; und
- die erste Ident-Kennzeichnung (ID-1-Label) unlösbar mit der Verbrauchseinheit (D) verbunden und zur eindeutigen Identifizierung der Verbrauchseinheit (D) eingerichtet und
- wobei die erste Ident-Kennzeichnung (ID-1-Label) mit einem, vorzugsweise nicht-zerstörungsfrei, lösbaren Verdeckungsmittel (150) teilweise oder vollständig verdeckt, vorzugsweise optisch verdeckt, ist.

2. Verbrauchseinheit (D) nach Anspruch 1, weiterhin umfassend:
eine einen zweiten Ident-Code (ID-2-Code) materiell ausbildende, zweite Ident-Kennzeichnung (ID-2-Label),
wobei die zweite Ident-Kennzeichnung (ID-2-Label) nicht-verdeckt, vorzugsweise optisch sichtbar ist, und zu einer zumindest vorübergehenden logistischen Verwendungsverfolgung dient und;
wobei die zweite Ident-Kennzeichnung (ID-2-Label) ein Teil des Verdeckungsmittels (150) ist.

3. Verfahren zur Kennzeichnung einer Verbrauchseinheit (D) für eine Verbrauchsanwendung eines Verbrauchsprodukts (100), vorzugsweise zum Verbrauch in einem Medizinapparat (200), zur Sicherung gegen Manipulation oder Missbrauch durch unberechtigte Dritte, mit den Schritten:
- informationstechnisches Generieren (S101) eines ersten Ident-Codes (ID-1-Code) und Speichern durch einen Hersteller (1) des Verbrauchsproduktes (100);
- Kennzeichnen (S103) der Verbrauchseinheit (D) mit einer, den ersten Ident-Code (ID-1-Code) materiell ausbildenden, ersten Ident-Kennzeichnung (ID-1-Label); und damit einhergehend
- informationstechnisches Zuordnen (S104) von einer Produkttype (Type) und/oder Produktverbrauchseigenschaft der Verbrauchseinheit (D) zu dem ersten Ident-Code (ID-1-Code) zur Bildung einer ersten Datenrelation (ID-1-Code/Type) und Speichern durch den Hersteller (1); und
- Verdecken (S107) der ersten Ident-Kennzeichnung (ID-1-Label) mit einem Verdeckungsmittel (150), wobei
∘ die erste Ident-Kennzeichnung (ID-1-Label) teilweise oder vollständig verdeckt, vorzugsweise optisch verdeckt ist; und
∘ das Verdeckungsmittel (150) lösbar und vorzugsweise nicht-zerstörungsfrei lösbar von der ersten Ident-Kennzeichnung (ID-1-Label) aufgebracht wird.

4. Verfahren zur Kennzeichnung nach Anspruch 3, weiter umfassend die Schritte:
- informationstechnisches Generieren (S102) eines zweiten Ident-Codes (ID-2-Code) und Speichern durch den Hersteller (1);
- Kennzeichnen (S105) der Verbrauchseinheit (D) mit einer, den zweiten Ident-Code (ID-2-Code) materiell ausbildenden, zweiten Ident-Kennzeichnung (ID-2-Label), wobei die zweite Ident-Kennzeichnung (ID-2-Label) nicht-verdeckt, vorzugsweise optisch sichtbar, aufgebracht wird;
- informationstechnisches Zuordnen (S106) des zweiten Ident-Codes (ID-2-Code) zu dem ersten Ident-Code (ID-1-Code) zur Bildung einer zweiten Datenrelation (ID-1-Code/ID-2-Code) und Speichern durch den Hersteller (1).

5. Verfahren zur Kennzeichnung nach Anspruch 2, wobei die zweite Ident-Kennzeichnung (ID-2-Label) zum Kennzeichnen (S105) der Verbrauchseinheit (D) ein Teil des Verdeckungsmittels(150) ist und damit auch das teilweise oder vollständige Verdecken (S107) der ersten Ident-Kennzeichnung (ID-1-Label) bewirkt.

6. Verfahren zur Kennzeichnung nach einem der Ansprüche 3 bis 5, wobei das Kennzeichnen (S103) mit der ersten Ident-Kennzeichnung (ID-1-Label) erfolgt:
- durch eine nicht-zerstörungsfrei lösbare Verbindung; und/oder
- mittels Stanzen, Lasern, Drucken, Prägen, Beschichten, Coating, Inlay-Verfahren, festem Verkleben, 3D-Printing, Versiegeln; und/oder
- in einer Qualitätssicherungsstufe der Herstellung der Verbrauchseinheit (D); und/oder
- in einer Verpackungsstufe der Herstellung der Verbrauchseinheit (D); und/oder
- auf einer jeweiligen Umverpackung der Verpackungseinheit (D); und/oder
- optisch sichtbar unter einer transparenten jeweiligen Umverpackung der Verpackungseinheit (D); und/oder
- durch eine zumindest in einem original-verpackten und/oder original-verschlossenen Zustand der Verbrauchseinheit (D) unlösbare Verbindung; und/oder
- in einem zumindest im dem original-verpackten und/oder original-verschlossenen Zustand verdeckten und/oder von außen nicht zugänglichen Bereich der Verbrauchseinheit (D), wie innenliegend in einer Verschlusskappe.

7. Verfahren zur Kennzeichnung nach einem der Ansprüche 3 bis 6, wobei das Verdecken (S107) mit dem Verdeckungsmittel (150) und/oder das Kennzeichnen (S105) mit der zweiten Ident-Kennzeichnung (ID-2-Label) erfolgt:
- durch eine für die erste Ident-Kennzeichnung zerstörungsfrei lösbare Verbindung; und/oder
- mittels reversiblen Oberflächenverfahren und/oder Mehrschichtverfahren, vorzugsweise mittels Aufbringen einer Aufkleberdeckschicht und/oder einer abrubbelbaren Deckschicht und/oder einer Trennzwischenschicht und/oder auf eine lösbare Haftschicht und/oder mittels Drucken mit reversibler Druckfarbe; und/oder
- in einer Qualitätssicherungsstufe der Herstellung der Verbrauchseinheit (D); und/oder
- in einer Verpackungsstufe der Herstellung der Verbrauchseinheit (D); und/oder
- in-line im direkten Anschluss zu und/oder in einer integralen Verfahrensstufe mit dem Schritt Kennzeichnen (S103) mit der ersten Ident-Kennzeichnung (ID-1-Label); und/oder
- auf einer jeweiligen Umverpackung der Verpackungseinheit (D); und/oder
- optisch sichtbar unter einer transparenten jeweiligen Umverpackung der Verpackungseinheit (D); und/oder
- hinsichtlich der zweiten Ident-Kennzeichnung (ID-2-Label) in Form eines zu Logistikzwecken verwendbaren Labels, wie beispielsweise einem RFID-Label, insbesondere einem zum Speichern einer Transaktionskette eingerichteten Label; und/oder
- im Falle des nicht mit der zweiten Ident-Kennzeichnung (ID-2-Label) zusammenfallenden Verdeckungsmittels (150), hinsichtlich der zweiten Ident-Kennzeichnung (ID-2-Label) nur auf einer Großpackung mit mehreren Verbrauchseinheiten (D).

8. Verfahren zur Freigabe einer Verbrauchsanwendung einer Verbrauchseinheit (D) nach Anspruch 1 oder 2 in einer zugehörigen Verbrauchsvorrichtung (200), vorzugsweise in einem Medizinapparat (200), mit Sicherung gegen Manipulation oder Missbrauch durch unberechtigte Dritte, umfassend die Schritte:
- Entfernen (S108) des Verdeckungsmittels (150) von der Verbrauchseinheit (D) an dem Verbrauchsort, wobei die erste Ident-Kennzeichnung (ID-1-Label) erkennbar, vorzugsweise optisch sichtbar, freigelegt wird; und
- Autorisieren (S109) des Verbrauches und/oder eines jeweilig zugehörigen Verbrauchszweckes der Verbrauchseinheit (D), wobei zumindest der erste Ident-Code (ID-1-Code) erkannt wird.

9. Verfahren zur Freigabe nach Anspruch 8, wobei in dem Schritt des Autorisierens (S109) weiterhin der zweite Ident-Code (ID-2-Code) erkannt wird, vorzugsweise vorab zu der und/oder im selben Zuge mit der Erkennung des ersten Ident-Codes (ID-1-Code).

10. Verfahren zur Freigabe nach Anspruch 8 oder 9, wobei zumindest der erste Ident-Code (ID-1-Code) durch den Verbraucher (2) und/oder den Anwender (3) visuell gelesen bzw. mittels eines separaten Code-Lesegeräts (250) erkannt und an die Verbrauchsvorrichtung (200) weitergegeben wird und/oder durch die Verbrauchsvorrichtung (200) automatisch erkannt wird.

11. Verfahren zur Freigabe nach einem der Ansprüche 8 bis 10, wobei aufgrund einer Verbindung des Verdeckungsmittels (150), insbesondere der zweiten Ident-Kennzeichnung (ID-2-Label), mit der Verbrauchseinheit (D) das Entfernen (S108) des Verdeckungsmittels (150) im Zuge eines Verbrauchs der Verbrauchseinheit (D) mitbewirkt wird, vorzugsweise im Zuge des Öffnens der Verbrauchseinheit (D) und/oder eines Anschließens der Verbrauchseinheit (D) an die Verbrauchsvorrichtung (200).

12. Verfahren zur Freigabe nach einem der Ansprüche 8 bis 11, als weiteren Schritt eine Sperrwirkung (S110) der Verbrauchseinheit (D) für einen Verbrauch umfassend, vorzugsweise nach einem Ablehnen bzw. Fehlschlagen des Autorisierens (S109) betreffend der Nicht-Einhaltung eines für den Verbraucher (2) und/oder den Anwender (3) zuvor hinterlegten zulässigen Verbrauchszweckes, auf Basis einer Überprüfung der Produkttype (Type) und/oder Produktverbrauchseigenschaft der Verbrauchseinheit (D); und/oder
einer der Verbrauchseinheit (D) zugeordneten Zeitobergrenze, vorzugsweise auf Basis einer Haltbarkeitsdauer und/oder eines Zeitfensters ab Erreichen des Verbrauchsorts und/oder ab Erkennen des ersten Ident-Codes (ID-1-Code); und/oder
einer Kompatibilität von technischen Anforderungen zum ordnungsgemäßen Verbrauch der Verbrauchseinheit (D) mit den technischen Daten der Verbrauchsvorrichtung (200),
wobei im Falle einer Sperrwirkung vorzugsweise weiter eine Information (S111) über einen auslösenden Grund und/oder zu einer Problemlösung durch den Hersteller (1) erfolgt.

13. Verfahren zur Systemsteuerung zur Ausführung eines Verfahrens zur Freigabe nach einem der Ansprüche 8 bis 11 in Kombination mit einem Verfahren zur Kennzeichnung nach einem der Ansprüche 3 bis 7, wobei der Schritt des Autorisierens (S109) mittels informationstechnischem Abgleichen (S113) des an dem Verbrauchsort erkannten ersten Ident-Codes (ID-1-Code) mit dem generierten und durch den Hersteller (1) gespeicherten (S101) ersten Ident-Code (ID-1-Code) erfolgt, vorzugsweise indem
- das Abgleichen (S113) mit einer in der Verbrauchsvorrichtung (200) zuvor hinterlegten Kopie des generierten ersten Ident-Codes (ID-1-Code) erfolgt; und/oder
- das Abgleichen (S113) mit einer bei dem Verbraucher (2) und/oder dem Anwender (3) zuvor hinterlegten Kopie des generierten ersten Ident-Codes (ID-1-Code) erfolgt; und/oder
- das Abgleichen (S113) mit einer bei einer dezentralen Datenbank eines berechtigten weiteren Teilnehmers (4) zuvor hinterlegten Kopie des generierten ersten Ident-Codes (ID-1-Code) erfolgt; und/oder
- das Abgleichen (S113) und/oder Übermitteln der zuvor hinterlegten Kopie mittels Fernkommunikation über eine Schnittstelle zu einer Datenbank des Herstellers (1) zur Verwaltung zumindest des ersten Ident-Codes (ID-1-Code) erfolgt.

14. Verfahren zur Systemsteuerung nach Anspruch 13, wobei der Schritt des Autorisierens (S109) als weiteren Schritt eine informationstechnische Verwendung (S115) der zweiten Datenrelation (ID-1-Code/ID-2-Code) zur Berücksichtigung einer bis zum Verbrauchsort gesammelten Information und/oder Transaktionskette, umfasst.

15. Verfahren zur Systemsteuerung nach Anspruch 13 oder 14, wobei der Schritt des Autorisierens (S109) als weiteren Schritt eine informationstechnische Verwendung (S114) der ersten Datenrelation (ID-1-Code/Type) zur Berücksichtigung der Produkttype (Type) und/oder Produktverbrauchseigenschaft der Verbrauchseinheit (D) umfasst.

16. Verfahren zur Systemsteuerung nach Ansprüchen 13 bis 15, wobei ein informationstechnischer Schritt in zumindest einer Transaktionskette, vorzugsweise in einer Blockkette mit verknüpften Transaktionen in einem zur Gewährleistung von Dokumentationssicherheit zwischen mehreren verteilten Teilnehmern (1, 2, 3, 4) eingerichteten Datenbankmanagement-Steuersystem (300), verarbeitet und/oder hinterlegt wird, insbesondere betreffend einen oder mehrere der Schritte:
- Code-Generieren (S101, S102); Speichern durch den Hersteller (1), vorzugsweise von Daten aus Produktion und Logistik; Zuordnen (S104) zur Bildung der ersten Datenrelation (ID-1-Code/Type), Zuordnen (S106) zur Bildung der zweiten Datenrelation (ID-1-Code/ID-2-Code); Autorisieren (S109); Abgleichen (S113); Überprüfen; Verwendung (S114) der ersten Datenrelation (ID-1-Code/Type), Verwendung (S115) der zweiten Datenrelation (ID-1-Code/ID-2-Code); Sperrwirkung (S110); und/oder
- Ausgeben einer Information (S111), wobei im bevorzugten Falle eines Datenbankmanagement-Steuersystems (300) das Verfahren insbesondere zum Zwecke der Dokumentation einer Verbrauchsverwendung eines Teilnehmers (1, 2, 3, 4) und/oder zum Zwecke einer Überwachung eingerichtet ist.

17. Steuersystem (400), welches zur Ausführung eines Verfahrens zur Systemsteuerung nach Ansprüchen 13 bis 16 eingerichtet ist, umfassend:
- ein Steuergerät; und
- eine Kennzeichnungsvorrichtung (199), eingerichtet zum gleichzeitigen und/oder separaten Aufbringen zumindest der ersten Ident-Kennzeichnung (ID-1-Label) und/oder des Verdeckungsmittels (150) an dem Ort des Herstellers (1); und/oder
- die Verbrauchsvorrichtung (200), zum Verbrauch des Verbrauchsprodukts (100) an dem Ort des Verbrauchers (2) und/oder des Anwenders (3).

18. Computerprogramm, geladen in einen digitalen Speicher eines Computers, welches ein Verfahren nach einem der Ansprüche 3 bis 16 ausführt und/oder ein Steuersystem (400) nach Anspruch 17 implementiert.

19. Sicherheitselement (110) ausgestaltet zum Aufbringen auf eine Verbrauchseinheit (D) für eine Verbrauchsanwendung eines Verbrauchsprodukts (100), vorzugsweise zum Verbrauch in einem Medizinapparat (200), und eingerichtet zur Sicherung der Verbrauchseinheit (D) gegen Manipulation oder Missbrauch durch unberechtigte Dritte, umfassend:
eine Kombination aus einer einen ersten Ident-Code (ID-1-Code) materiell ausbildenden ersten Ident-Kennzeichnung (ID-1-Label) und einer einen zweiten Ident-Code (ID-2-Code) materiell ausbildenden zweiten Ident-Kennzeichnung (ID-2-Label), wobei
die erste Ident-Kennzeichnung (ID-1-Label) eingerichtet ist, eine unlösbare Verbindung zu der Verbrauchseinheit (D) auszubilden und eine eindeutige Identifizierung der Verbrauchseinheit (D), wie hinsichtlich einer Produkttype (Type) und/oder Produktverbrauchseigenschaft der Verbrauchseinheit (D), zu stellen;
die erste Ident-Kennzeichnung (ID-1-Label) mit einem, vorzugsweise nicht-zerstörungsfrei lösbaren, Verdeckungsmittel (150) teilweise oder vollständig verdeckt, vorzugsweise optisch verdeckt, ist; und
die zweite Ident-Kennzeichnung (ID-2-Label) lösbar und zu einer vorübergehenden Verwendung, wie logistischer Nachverfolgbarkeit, eingerichtet ist;
vorzugsweise die zweite Ident-Kennzeichnung (ID-2-Label) identisch zu dem Verdeckungsmittel (150) ist; und/oder
vorzugsweise das Verdeckungsmittel (150) nicht-zerstörungsfrei lösbar ist.
